(19) Europäisches Patentamt
European Patent Office
Office européen des brevets

(11) Veröffentlichungsnummer: **0 611 660 A2**

# (12) EUROPÄISCHE PATENTANMELDUNG

(21) Anmeldenummer: **94102222.0**

(22) Anmeldetag: **14.02.94**

(51) Int. Cl.[5]: **B41J X/**, C07D 471/04, C07D 471/16, C07D 487/16, C07D 413/06, C07D 403/06, C07D 401/12, C07D 401/10, C07D 401/14, C07D 401/06, A61K 31/415

(30) Priorität: **16.02.93 DE 4304650**

(43) Veröffentlichungstag der Anmeldung: **24.08.94 Patentblatt 94/34**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach (DE)**

(72) Erfinder: **Austel, Volkhard, Prof. Dr. Dipl.-Chem.**
**Kapellenweg 7**
**D-88440 Biberach (DE)**
Erfinder: **Pieper, Helmut, Dr. Dipl.-Chem.**
**Kapellenweg 5**
**D-88400 Biberach (DE)**
Erfinder: **Himmelsbach, Frank, Dr. Dipl.-Chem.**
**Ahornweg 16**
**D-88441 Mittelbiberach (DE)**
Erfinder: **Linz, Günther, Dr. Dipl.-Chem.**
**Erlenweg 8**
**D-88441 Mittelbiberach (DE)**
Erfinder: **Müller, Thomas, Dr. Arzt und Dipl.-Chem.**
**Alter Postplatz 17**
**D-88400 Biberach (DE)**
Erfinder: **Weisenberger, Johannes, Dr. Dipl.-Chem.**
**Haydnweg 5**
**D-88400 Biberach (DE)**
Erfinder: **Guth, Brian, Dr. Phys.**
**Am Schlegelberg 24**
**D-88447 Warthausen (DE)**

## (54) Einstellbare Blattwellvorrichtung mit Förderklemme.

(57) Die Erfindung betrifft kondensierte 5-gliedrige Heterocyclen der allgemeinen Formel

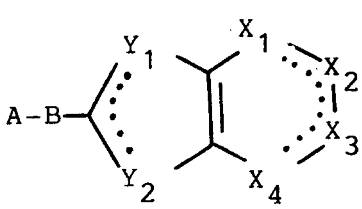

, (I)

in der

$R_a$, $R_b$, X und Y wie im Anspruch 1 definiert sind, deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

EP 0 611 660 A2

Die Erfindung betrifft kondensierte 5-gliedrige Heterocyclen der allgemeinen Formel

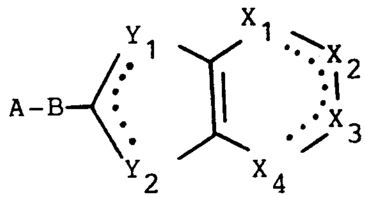

, (I)

deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel, deren Verwendung und Verfahren zu ihrer Herstellung.

In der obigen allgemeinen Formel I bedeutet

A eine Aminoalkyl-, Amidino- oder Guanidinogruppe, in denen jeweils an einem der Stickstoffatome ein Wasserstoffatom durch eine Hydroxy-, Alkyl-, Alkoxycarbonyl- oder Phenylalkoxycarbonylgruppe ersetzt sein kann,

eine gegebenenfalls im Kohlenstoffgerüst durch eine oder zwei Alkylgruppen und am Stickstoffatom durch den Rest $R_a$ substituierte Piperidinylgruppe, in der

$R_a$ ein Wasserstoffatom, eine Alkyl- oder Phenylalkylgruppe, eine Alkoxycarbonylgruppe mit insgesamt 2 bis 5 Kohlenstoffatomen, eine Phenylalkoxycarbonylgruppe oder eine $R_1$-CO-($R_2$CH)-O-CO-Gruppe darstellt, in welcher

$R_1$ eine Alkylgruppe und

$R_2$ ein Wasserstoffatom, eine Alkyl- oder Phenylgruppe darstellen,

und zusätzlich eine $>$CH- Einheit in 4-Stellung durch ein Stickstoffatom ersetzt sein kann,

eine Imidazolylgruppe oder

eine Pyridylgruppe, die falls das heterocyclische System eine Benzoaxazolgruppe darstellt, nicht über die 2-Stellung an den Rest B gebunden sein kann,

B eine geradkettige oder verzweigte Alkylengruppe mit 1 bis 5 Kohlenstoffatomen,

eine -Alkylen-O-, -O-alkylen-, -Alkylen-S-, -S-alkylen-, -Alkylen-$NR_3$-, -$NR_3$-alkylen-, -CO-$NR_3$- oder -$NR_3$-CO-Gruppe, in denen

$R_3$ ein Wasserstoffatom, eine Alkyl- oder Phenylalkylgruppe darstellt,

eine Cyclohexylengruppe oder auch, wenn

A eine gegebenenfalls im Kohlenstoffgerüst durch eine oder zwei Alkylgruppen und am Stickstoffatom durch den Rest $R_a$ substituierte Piperidinylgruppe darstellt, wobei $R_a$ wie oben definiert ist und eine $>$CH-Einheit in 4-Stellung durch ein Stickstoffatom ersetzt sein kann, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe, die im Phenylkern durch Fluor-, Chlor- oder Bromatome, durch Alkyl- oder Alkoxygruppen mono- oder disubstituiert sein kann, wobei die Substituenten gleich oder verschieden sein können,

einer der Reste $X_1$ bis $X_4$ eine F-E-D-C$\ll$ oder F-E-D-N$<$ Gruppe, in denen

D eine geradkettige oder verzweigte Alkylengruppe mit 1 bis 6 Kohlenstoffatomen, eine Alkenylengruppe mit 2 bis 6 Kohlenstoffatomen, ein Sauerstoff- oder Schwefelatom, eine -CO-, -SO-, -$SO_2$-, -CO-$NR_3$- oder -$NR_3$-CO-Gruppe oder eine über das Stickstoffatom an den Rest E gebundene -$SO_2$-$NR_3$-Gruppe, wobei $R_3$ wie vorstehend erwähnt definiert ist,

E eine Bindung oder eine geradkettige oder verzweigte Alkylengruppe mit 1 bis 5 Kohlenstoffatomen und

F eine Carboxygruppe, eine Alkoxycarbonylgruppe mit insgesamt 2 bis 7 Kohlenstoffatomen, eine Phenylalkoxycarbonylgruppe, eine Cycloalkoxycarbonyl- oder Cycloalkylalkoxycarbonylgruppe mit jeweils 3 bis 7 Kohlenstoffatomen im Cycloalkylteil, eine Sulfo-, Phosphono-, O-Alkyl-phosphono-, O,O-Dialkylphosphono- oder Tetrazol-5-yl-Gruppe darstellen,

ein zweiter der Reste $X_1$ bis $X_4$ ein Stickstoffatom, eine $R_b$-C$<$ , $R_c$-N$<$ oder Carbonylgruppe, in denen

$R_b$ ein Wasserstoff-, Fluor-, Chlor- oder Bromatom, eine Alkyl-, Hydroxy-, Alkoxy-, Phenylalkoxy-, Amino-, Alkylamino- oder Dialkylaminogruppe, wobei jeder Alkylteil in der vorstehend erwähnten Gruppen jeweils 1 bis 6 Kohlenstoffatome enthalten kann, und

$R_c$ ein Wasserstoffatom, eine gegebenenfalls durch eine Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl-, Pyrrolidino-, Piperidino-, Morpholino-, Piperazino- oder N-Alkyl-piperazinogruppe substituierte Alkylgruppe mit 1 bis 6 Kohlenstoffatomen oder

eine Phenylalkylgruppe mit 1 bis 5 Kohlenstoffatomen im Alkylteil, die im Phenylkern durch Fluor-, Chlor- oder Bromatome, durch Alkyl- oder Alkoxygruppen mono- oder disubstituiert sein kann, wobei die Substituenten gleich oder verschieden sein können, darstellen,

und die verbleibenden Reste der Reste $X_1$ bis $X_4$ jeweils ein Stickstoffatom, eine $R_b$-C$\ll$ oder Carbonylgruppe, wobei $R_b$ wie vorstehend erwähnt definiert ist und nicht mehr als zwei der Reste $X_1$ bis $X_4$ Carbonylgruppen darstellen können und nicht mehr als 3 der Reste $X_1$ bis $X_4$ ein im Ring befindliches Stickstoffatom enthalten können,

einer der Reste $Y_1$ oder $Y_2$ ein Stickstoffatom oder eine Methingruppe und

der andere der Reste $Y_1$ oder $Y_2$ ein Sauerstoffatom oder eine $R_d$-N$<$ Gruppe, in der

$R_d$ ein Wasserstoffatom, eine gegebenenfalls durch eine Hydroxy-, Alkoxy-, Amino-, Alkylamino-, Dialkylamino-, Pyrrolidino-, Piperidino-, Morpholino-, Piperazino- oder N-Alkyl-piperazinogruppe substituierte Alkylgruppe mit 1 bis 6 Kohlenstoffatomen,

eine Phenylalkylgruppe mit 1 bis 5 Kohlenstoffatomen im Alkylteil, die im Phenylkern durch Fluor-, Chlor- oder Bromatome, durch Alkyl- oder Alkoxygruppen mono- oder disubsitutiert sein kann und die Substituenten gleich oder verschieden sein können,

eine Cycloalkylgruppe mit 3 bis 7 Kohlenstoffatomen oder, wenn die Gruppen $R_c$-N$<$ und $R_d$-N$<$ an dasselbe Kohlenstoffatom gebunden sind,

$R_d$ zusammen mit $R_c$ eine geradkettige oder verzweigte Alkylengruppe mit 2 bis 5 Kohlenstoffatomen darstellen,

wobei, soweit nichts anderes erwähnt wurde, die vorstehend erwähnten Alkyl-, Alkylen- und Alkoxyteile jeweils 1 bis 3 Kohlenstoffatome enthalten können.

Bevorzugte Verbindungen der obigen allgemeinen Formel I sind jedoch diejenigen, in denen

A eine gegebenenfalls durch eine Hydroxy- oder Alkoxycarbonylgruppe substituierte Amidinogruppe, eine Aminoalkyl- oder Benzyloxycarbonylaminoalkylgruppe,

eine gegebenenfalls im Kohlenstoffgerüst durch eine Alkylgruppe und am Stickstoffatom durch den Rest $R_a$ substituierte Piperidinylgruppe, in der

$R_a$ ein Wasserstoffatom, eine Alkyl-, Benzyl-, Alkoxycarbonyl-, Benzyloxycarbonyl- oder $R_1$-CO-$CH_2$-O-CO-Gruppe darstellt, in welcher

$R_1$ eine Alkylgruppe darstellt,

und zusätzlich eine $>$CH- Einheit in 4-Stellung durch ein Stickstoffatom ersetzt sein kann,

eine Imidazolylgruppe oder

eine Pyridylgruppe, die falls das heterocyclische System eine Benzoxazolgruppe darstellt, nicht über die 2-Stellung an den Rest B gebunden sein kann,

B eine geradkettige oder verzweigte Alkylengruppe mit 1 bis 5 Kohlenstoffatomen,

eine -Alkylen-O-, -O-alkylen-, -Alkylen-S-, -S-alkylen-, -Alkylen-$NR_3$-, -$NR_3$-alkylen-, -CO-$NR_3$- oder -$NR_3$-CO-Gruppe, in denen

$R_3$ ein Wasserstoffatom oder eine Alkylgruppe darstellt,

eine Cyclohexylengruppe oder auch,

wenn A eine gegebenenfalls im Kohlenstoffgerüst durch eine Alkylgruppe und am Stickstoffatom durch den Rest $R_a$ substituierte Piperidinylgruppe darstellt, wobei $R_a$ wie oben definiert ist und eine $>$CH-Einheit in 4-Stellung durch ein Stickstoffatom ersetzt sein kann, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C$\ll$ oder F-E-D-N$<$ Gruppe, in denen

D eine geradkettige oder verzweigte Alkylengruppe mit 1 bis 6 Kohlenstoffatomen, eine Alkenylengruppe mit 2 bis 4 Kohlenstoffatomen, ein Sauerstoff- oder Schwefelatom, eine -CO-, -SO-, -$SO_2$-, -CO-$NR_3$- oder -$NR_3$-CO-Gruppe oder eine über das N-Atom an den Rest E gebundene -$SO_2$-$NR_3$-Gruppe, wobei $R_3$ wie vorstehend erwähnt definiert ist,

E eine Bindung oder eine geradkettige oder verzweigte Alkylengruppe mit 1 bis 5 Kohlenstoffatomen und

F eine Carboxygruppe, eine Alkoxycarbonylgruppe mit insgesamt 2 bis 6 Kohlenstoffatomen oder eine

Cycloalkoxycarbonylgruppe mit insgesamt 6 bis 8 Kohlenstoffatomen, darstellen,

ein zweiter der Reste $X_1$ bis $X_4$ ein Stickstoffatom, eine $R_b$-C⪡ , $R_c$-N< oder Carbonylgruppe, in denen

$R_b$ ein Wasserstoff-, Chlor- oder Bromatom, eine Alkyl-, Hydroxy-, Alkoxy-,Amino-, Alkylamino- oder Dialkylaminogruppe und

$R_c$ ein Wasserstoffatom, eine gegebenenfalls durch eine Hydroxy-, Alkoxy-, Amino-, Alkylamino-, Dialkylamino-, Carboxy-, Alkoxycarbonyl-, Aminocarbonyl-, Pyrrolidino-, Piperidino-, Morpholino-, Piperazino- oder N-Alkyl-piperazinogruppe substituierte Alkylgruppe mit 1 bis 5 Kohlenstoffatomen oder

eine Phenylalkylgruppe mit 1 bis 5 Kohlenstoffatomen im Alkylteil, die im Phenylkern durch Alkoxygruppen mono- oder disubstituiert sein kann, wobei die Substituenten gleich oder verschieden sein können, darstellen,

und die verbleibenden Reste der Reste $X_1$ bis $X_4$ jeweils ein Stickstoffatom, eine $R_b$-C⪡ oder Carbonylgruppe, wobei $R_b$ wie vorstehend erwähnt definiert ist und nicht mehr als zwei der Reste $X_1$ bis $X_4$ Carbonylgruppen darstellen können und nicht mehr als 3 der Reste $X_1$ bis $X_4$ ein im Ring befindliches Stickstoffatom enthalten können,

einer der Reste $Y_1$ oder $Y_2$ ein Stickstoffatom und

der andere der Reste $Y_1$ oder $Y_2$ ein Sauerstoffatom oder eine $R_d$-N< Gruppe, in der

$R_d$ ein Wasserstoffatom, eine gegebenenfalls durch eine Hydroxy-, Alkoxy-, Amino-, Alkylamino-, Dialkylamino-, Pyrrolidino-, Piperidino-, Morpholino-, Piperazino- oder N-Alkyl-piperazinogruppe substituierte Alkylgruppe mit 1 bis 5 Kohlenstoffatomen,

eine Phenylalkylgruppe mit 1 bis 5 Kohlenstoffatomen im Alkylteil, die im Phenylkern durch Alkoxygruppen mono- oder disubsitutiert sein kann und die Substituenten gleich oder verschieden sein können,

eine Cycloalkylgruppe mit 3 bis 6 Kohlenstoffatomen oder, wenn die Gruppen $R_c$-N< und $R_d$-N< an dasselbe Kohlenstoffatom gebunden sind,

$R_d$ zusammen mit $R_c$ eine geradkettige oder verzweigte Alkylengruppe mit 2 bis 4 Kohlenstoffatomen darstellen,

bedeuten, wobei, soweit nichts anderes erwähnt wurde, die vorstehend erwähnten Alkyl-, Alkylen- und Alkoxyteile jeweils 1 bis 3 Kohlenstoffatome enthalten können,

deren Tautomere, deren Stereoisomere und deren Salze.

Besonders bevorzugte Verbindungen der obigen allgemeinen Formel I sind jedoch diejenigen, in denen

A eine gegebenenfalls durch eine Hydroxy- oder Alkoxycarbonylgruppe mit insgesamt 2 bis 4 Kohlenstoffatomen substituierte Amidinogruppe, eine Aminoalkyl- oder Benzyloxycarbonylaminoalkyl-gruppe

eine gegebenenfalls im Kohlenstoffgerüst durch eine Methylgruppe und am Stickstoffatom durch den Rest $R_a$ substituierte Piperidinylgruppe, in der

$R_a$ ein Wasserstoffatom, eine Methyl-, Benzyl-, Methoxycarbonyl-, Benzyloxycarbonyl- oder $CH_3$-CO-$CH_2$-O-CO-Gruppe darstellt,

und zusätzlich eine >CH- Einheit in 4-Stellung durch ein Stickstoffatom ersetzt sein kann,

eine 4-Pyridyl- oder 1-Imidazolylgruppe,

B eine geradkettige oder verzweigte Alkylengruppe mit 1 bis 4 Kohlenstoffatomen,

eine -Alkylen-O-, -O-alkylen-, -Alkylen-S-, -Alkylen-$NR_3$-,-$NR_3$-alkylen- oder -$NR_3$-CO-Gruppe, in denen

der Alkylenteil jeweils 1 oder 2 Kohlenstoffatome enthalten kann sowie der Alkylenteil der -Alkylen-S-Gruppe und das Stickstoffatom der -$NR_3$-CO-Gruppe jeweils mit dem Rest A verbunden ist und

$R_3$ ein Wasserstoffatom oder eine Methylgruppe darstellt,

eine 1,4-Cyclohexylengruppe oder auch,

wenn A eine gegebenenfalls im Kohlenstoffgerüst durch eine Methylgruppe und am Stickstoffatom durch den Rest $R_a$ substituierte Piperidinylgruppe darstellt, wobei $R_a$ wie oben definiert ist und eine >CH-Einheit in 4-Stellung durch ein Stickstoffatom ersetzt sein kann, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C⪡ oder F-E-D-N< Gruppe, in denen

D eine geradkettige oder verzweigte Alkylengruppe mit 1 bis 6 Kohlenstoffatomen, eine Alkenylengruppe mit 2 bis 4 Kohlenstoffatomen, ein Sauerstoff- oder Schwefelatom, eine -CO-, -SO-, -$SO_2$-, -CO-$NR_3$- oder -$NR_3$-CO-Gruppe oder eine über das Stickstoffatom an den Rest E gebundene -$SO_2$-$NR_3$-Gruppe, wobei $R_3$ wie vorstehend erwähnt definiert ist,

E eine Bindung oder eine geradkettige oder verzweigte Alkylengruppe mit 1 bis 4 Kohlenstoffatomen und

F eine Carboxygruppe, eine Alkoxycarbonylgruppe mit insgesamt 2 bis 6 Kohlenstoffatomen oder eine Cyclohexyloxycarbonylgruppe darstellen,
ein zweiter der Reste $X_1$ bis $X_4$ ein Stickstoffatom, eine $R_c$-N$\langle$ , $R_b$-C$\ll$ oder Carbonylgruppe, in denen
$R_b$ ein Wasserstoff- oder Chloratom, eine Hydroxy-, Methoxy-, Amino-,Methylamino- oder Dimethylaminogruppe und
$R_c$ ein Wasserstoffatom, eine gegebenenfalls durch eine Methoxy-, Carboxy-, Methoxycarbonyl- oder Aminocarbonylgruppe substituierte Alkylgruppe mit 1 bis 5 Kohlenstoffatomen oder
eine Phenylalkylgruppe mit 1 bis 5 Kohlenstoffatomen im Alkylteil, darstellen,
und die verbleibenden Reste der Reste $X_1$ bis $X_4$ jeweils ein Stickstoffatom, eine $R_b$-C$\langle$ oder Carbonylgruppe, wobei $R_b$ wie vorstehend erwähnt definiert ist und nicht mehr als zwei der Reste $X_1$ bis $X_4$ Carbonylgruppen darstellen können und nicht mehr als zwei der Reste $X_1$ bis $X_4$ ein im Ring befindliches Stickstoffatom enthalten können,
einer der Reste $Y_1$ oder $Y_2$ ein Stickstoffatom und
der andere der Reste $Y_1$ oder $Y_2$ ein Sauerstoffatom oder eine $R_d$-N$\langle$ Gruppe, in der
$R_d$ ein Wasserstoffatom, eine gegebenenfalls durch eine Hydroxy-, Methoxy-, Amino-, Methylamino-, Dimethylamino-, Morpholino-, Piperazino- oder N-Methyl-piperazinogruppe substituierte Alkylgruppe mit 1 bis 5 Kohlenstoffatomen,
eine Phenylalkylgruppe mit 1 bis 5 Kohlenstoffatomen im Alkylteil, die im Phenylkern durch Methoxygruppen mono- oder disubsitutiert sein kann,
eine Cycloalkylgruppe mit 3 bis 6 Kohlenstoffatomen oder, wenn die Gruppen $R_c$-N$\langle$ und $R_d$-N$\langle$ an dasselbe Kohlenstoffatom gebunden sind,
$R_d$ zusammen mit $R_c$ eine geradkettige oder verzweigte Alkylengruppe mit 2 oder 3 Kohlenstoffatomen darstellen, bedeuten,
insbesondere diejenigen Verbindung der obigen allgemeinen Formel I, in denen
A eine Amidino-, Aminoalkyl- oder Benzyloxycarbonylaminoalkylgruppe, in denen der Alkylteil jeweils 1 oder 2 Kohlenstoffatome enthalten kann, oder
eine am Stickstoffatom durch den Rest $R_a$ substituierte Piperidin-4-yl-Gruppe, in der
$R_a$ ein Wasserstoffatom, eine Benzyl-oder Benzyloxycarbonylgruppe darstellt,
B eine geradkettige oder verzweigte Alkylengruppe mit 1 bis 4 Kohlenstoffatomen,
eine -NH-CO-Gruppe, in der das Stickstoffatom der -NH-CO-Gruppe mit dem Rest A verbunden ist,
eine 1,4-Cyclohexylengruppe oder auch,
wenn A eine am Stickstoffatom durch den Rest $R_a$ substituierte Piperidin-4-yl-Gruppe darstellt, wobei $R_a$ wie vorstehend erwähnt definiert ist, oder wenn
$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,
oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,
eine Phenylengruppe,
einer der Reste $X_1$ bis $X_4$ eine F-E-D-C$\ll$ oder F-E-D-N$\langle$ -Gruppe, in denen
D eine geradkettige oder verzweigte Alkylengruppe mit 1 bis 4 Kohlenstoffatomen, eine Alkenylengruppe mit 2 oder 3 Kohlenstoffatomen, eine -CO- oder -CO-NH-Gruppe, wobei das Stickstoffatom der -CO-NH-Gruppe mit dem Rest E verknüpft ist,
E eine Bindung oder eine geradkettige oder verzweigte Alkylengruppe mit 2 bis 4 Kohlenstoffatomen und
F eine Carboxygruppe oder eine Alkoxycarbonylgruppe mit insgesamt 2 bis 5 Kohlenstoffatomen darstellen,
ein zweiter der Reste $X_1$ bis $X_4$, eine $R_c$-N$\langle$ oder $R_b$-C$\ll$ Gruppe, wobei
$R_b$ ein Wasserstoff- oder Chloratom und
$R_c$ ein Wasserstoffatom, eine Methyl- oder Ethylgruppe darstellen,
und die verbleibenden Reste der Reste $X_1$ bis $X_4$ jeweils ein Stickstoffatom, eine H-C$\ll$ oder Carbonylgruppe,
einer der Reste $Y_1$ oder $Y_2$ ein Stickstoffatom und
der andere der Reste $Y_1$ oder $Y_2$ eine $R_d$-N$\langle$ Gruppe, in der
$R_d$ ein Wasserstoffatom oder eine Alkylgruppe mit 1 bis 4 Kohlenstoffatomen oder $R_d$ zusammen mit $R_c$ eine geradkettige oder verzweigte Alkylengruppe mit 2 oder 3 Kohlenstoffatomen darstellen,
bedeuten, deren Tautomere, deren Stereoisomere und deren Salze.
Ganz besonders bevorzugte Verbindung der obigen allgemeinen Formel I sind diejenigen, in denen
A eine Amidino- oder Piperidin-4-yl-Gruppe,

B eine Ethylengruppe oder auch, wenn

A eine Piperidin-4-yl-Gruppe darstellt, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C≪ oder F-E-D-N< -Gruppe, in denen

D eine geradkettige oder verzweigte Alkylengruppe mit 2 bis 4 Kohlenstoffatomen, eine Ethenylengruppe, eine -CO- oder -CO-NH-Gruppe, wobei das Stickstoffatom der -CO-NH-Gruppe mit dem Rest E verknüpft ist,

E eine Bindung oder eine Ethylengruppe und

F eine Carboxygruppe oder eine Alkoxycarbonylgruppe mit insgesamt 2 bis 5 Kohlenstoffatomen darstellen,

ein zweiter der Reste $X_1$ bis $X_4$, eine $R_c$-N< oder $R_b$-C≪ Gruppe, wobei

$R_b$ ein Wassertoff- oder Chloratom und

$R_c$ ein Wasserstoffatom oder eine Methylgruppe darstellt,

und die verbleibenden Reste der Reste $X_1$ bis $X_4$ jeweils ein Stickstoffatom, eine H-C≪ oder Carbonylgruppe, einer der Reste $Y_1$ oder $Y_2$ ein Stickstoffatom und

der andere der Reste $Y_1$ oder $Y_2$ eine $R_d$-N< Gruppe, in der

$R_d$ eine Methylgruppe oder

$R_d$ zusammen mit $R_c$ eine n-Propylengruppe darstellen, bedeuten, deren Tautomere, deren Stereoisomere und deren Salze.

Als besonders bevorzugte Verbindungen der allgemeinen Formel I seien beispielsweise folgende genannt:

(a) 5-[(2-Carboxy-ethyl)-aminocarbonyl]-1-methyl-2-[2-(4-piperidinyl)-ethyl]-benzimidazol,
(b) 5-[(2-Carboxy-ethyl)-aminocarbonyl]-1-methyl-2-[(4-piperidinyl)-aminocarbonyl]-benzimidazol,
(c) 5-(4-Carboxy-1-oxo-butyl)-1-methyl-2-[2-(4-piperidinyl)-ethyl]-benzimidazol,
(d) 1-(4-Carboxy-butyl)-3-methyl-8-[2-(4-piperidinyl)ethyl]-xanthin,
(e) 1-(4-Carboxy-butyl)-3,9-dimethyl-8-[2-(4-piperidinyl)ethyl]-xanthin,
(f) 2-(4-Amidino-phenyl)-9-(4-carboxy-butyl)-8,10-dioxo-5,6-dihydro-4H,9H-pyrimido[1,2,3-cd]purin,
(g) 5-[(2-Methoxycarbonyl-ethyl)-aminocarbonyl]-1-methyl-2-[2-(4-piperidinyl)-ethyl]-benzimidazol und deren Salze.

Erfindungsgemäß erhält man die neuen Verbindungen beispielsweise nach folgenden Verfahren:

a) Zur Herstellung von Verbindungen der allgemeinen Formel I, in der F eine Carboxygruppe darstellt: Abspaltung eines Schutzrestes von einer Verbindung der allgemeinen Formel

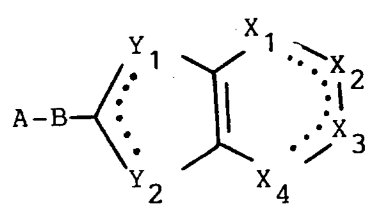

,(II)

in der

A, B, $X_1$ bis $X_4$, $Y_1$ und $Y_2$ mit der Maßgabe wie eingangs definiert sind, daß F eine Alkoxycarbonylgruppe mit insgesamt 2 bis 7 Kohlenstoffatomen, eine Phenylalkoxycarbonylgruppe mit 1 bis 3 Kohlenstoffatomen im Alkoxyteil, eine Cycloalkoxycarbo nyl- oder Cycloalkylalkoxcarbonylgruppe mit jeweils 3 bis 7 Kohlenstoffatomen im Cycloalkylteil und 1 bis 3 Kohlenstoffatomen im Alkoxyteil oder eine durch einen abspaltbaren Rest geschützte Carboxylgruppe wie die Trimethylsilyloxycarbonyl-, Methoxybenzyloxycarbonyl-, 2,4-Dimethoxybenzyloxycarbonyl- oder Tetrahydropyranyloxycarbonylgruppe darstellt,mittels Hydrolyse, Hydrogenolyse oder Thermolyse.

Die Hydrolyse wird zweckmäßigerweise entweder in Gegenwart einer Säure wie Salzsäure, Bromwasserstoffsäure, Schwefelsäure, Phosphorsäure, Essigsäure, Essigsäure/Salzsäure, Trichloressigsäure oder Trifluoressigsäure oder in Gegenwart einer Base wie Lithiumhydroxid, Natriumhydroxid oder Kaliumhydroxid in einem geeigneten Lösungsmittel wie Wasser, Methanol, Wasser/Methanol, Ethanol, Wasser/Ethanol, Wasser/Isopropanol, Wasser/Tetrahydrofuran oder Wasser/Dioxan bei Temperaturen

zwischen -10 °C und 120 °C, z.B. bei Temperaturen zwischen Raumtemperatur und der Siedetemperatur des Reaktionsgemisches, durchgeführt.

Bei der sauren Hydrolyse können je nach den angewandten Bedingungen auch andere in einer Verbindung der Formel II gegebenenfalls vorhandene hydrolytisch abspaltbare Gruppen wie die Acetyl-, Trifluoracetyl-, Benzoyl-, tert.Butyloxycarbonyl- oder Benzyloxycarbonylgruppe gleichzeitig abgespalten werden.

Bedeutet beispielsweise F die tert. Butyloxycarbonylgruppe, so kann die tert. Butylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet beispielsweise F die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe oder eine Benzyloxygruppe zur Hydroxygruppe und eine Benzylamino- oder Benzyloxycarbonylaminogruppe zu einer Aminogruppe mitreduziert werden. Außerdem können gleichzeitig C=C-Doppelbindung zu Einfachbindungen aufhydriert werden.

b) Zur Herstellung von Verbindungen der allgemeinen Formel I, in der $R_a$ ein Wasserstoffatom darstellt: Abspaltung eines Schutzrestes von einer Verbindung der allgemeinen Formel

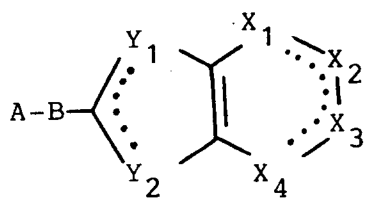

, (III)

in der

A, B, $X_1$ bis $X_4$, $Y_1$ und $Y_2$ mit der Maßgabe wie eingangs definiert sind, daß $R_a$ eine Alkoxycarbonylgruppe mit insgesamt 2 bis 5 Kohlenstoffatomen, eine Phenylalkoxycarbonylgruppe mit 1 bis 3 Kohlenstoffatomen im Alkoxyteil, eine $R_1$-CO-($R_2$CH)-O-CO-Gruppe, in der $R_1$ und $R_2$ wie eingangs definiert sind, oder einen abspaltbaren Schutzrest für eine Iminogruppe wie die Acetyl-, Trifluoracetyl-, Benzoyl-, Benzyl-, Methoxybenzyl-oder 2,4-Dimethoxybenzylgruppe darstellt, mittels Hydrolyse, Hydrogenolyse oder Thermolyse.

Die Hydrolyse wird zweckmäßigerweise entweder in Gegenwart einer Säure wie Salzsäure, Bromwasserstoffsäure, Schwefelsäure, Phosphorsäure, Essigsäure, Essigsäure/Salzsäure, Trichloressigsäure oder Trifluoressigsäure oder in Gegenwart einer Base wie Lithiumhydroxid, Natriumhydroxid oder Kaliumhydroxid in einem geeigneten Lösungsmittel wie Wasser, Methanol, Wasser/Methanol, Ethanol, Wasser/Ethanol, Wasser/Isopropanol, Wasser/Tetrahydrofuran, Ether/Dioxan oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bei der sauren Hydrolyse können je nach den angewandten Bedingungen auch andere in einer Verbindung der Formel III gegebenenfalls vorhandene hydrolytisch abspaltbare Gruppen wie Alkoxycarbonyl- oder Phenylalkoxycarbonylgruppen gleichzeitig abgespalten werden.

Bedeutet beispielsweise $R_a$ die tert. Butyloxycarbonylgruppe, so kann die tert. Butyloxycarbonylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methanol, Methylenchlorid, Chloroform, Benzol, Toluol, Tetrahydrofuran, Dioxan, Ether/Dioxan oder Ether/Dioxan/Methanol vorzugsweise bei Temperaturen zwischen -10 °C und 120 °C,

z.B. bei Temperaturen zwischen 0 und 60 ° C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 ° C und 100 ° C, abgespalten werden.

Bedeutet beispielsweise $R_a$ die Benzyl- oder Benzyloxycarbonylgruppe, so kann die Benzyl- oder Benzyloxycarbonylgruppe hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe oder eine Benzyloxygruppe zur Hydroxygruppe und eine Benzylaminogruppe zu einer Aminogruppe mitreduziert werden. Außerdem können gleichzeitig vorhandene C = C-Doppelbindungen zu Einfachbindungen aufhydriert werden.

c) Zur Herstellung von Verbindungen der allgemeinen Formel I, in der einer der Reste $Y_1$ oder $Y_2$ ein Stickstoffatom und der andere der Reste $Y_1$ oder $Y_2$ eine $R_d$-N< Gruppe oder, wenn die Gruppen $R_c$-N< und $R_d$-N< an dasselbe Kohlenstoffatom gebunden sind, $R_c$ und $R_d$ zusammen auch eine n-Alkylengruppe mit 2 bis 5 Kohlenstoffatomen darstellen:

Cyclisierung einer gegebenenfalls im Reaktionsgemisch gebildeten Verbindung der allgemeinen Formel

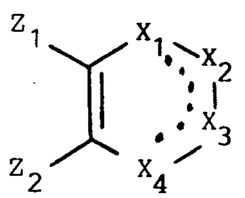

, (IV)

in der

$X_1$ bis $X_4$ wie eingangs definiert sind,

einer der Reste $Z_1$ oder $Z_2$ eine A-B-CO-$NR_{d1}$-Gruppe und der andere der Reste $Z_1$ oder $Z_2$ eine $HNR_{d2}$-Gruppe darstellen, in denen A und B wie eingangs definiert sind, einer der Reste $R_{d1}$ oder $R_{d2}$ ein Wasserstoffatom und der andere der Reste $R_{d1}$ oder $R_{d2}$ die für $R_d$ eingangs erwähnten Bedeutungen besitzt, und gegebenenfalls anschließende Abspaltung eines verwendeten - Schutzrestes.

Die Cyclisierung wird gegebenenfalls in einem Lösungsmittel oder Lösungsmittelgemisch wie Methanol, Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Diphenylether oder Tetralin oder in einem Überschuß des zur Herstellung der Verbindung der allgemeinen Formel IV verwendeten Acylierungsmittel, z.B. in der entsprechenden Säure oder dem entsprechenden Nitril, Anhydrid, Säurehalogenid, Ester oder Amid, beispielsweise bei Temperaturen zwischen 0 und 250 ° C, vorzugsweise jedoch bei Temperaturen zwischen 100 und 250 ° C, besonders vorteilhaft jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Natriumhydroxid, Kaliumethylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel IV im Reaktionsgemisch durch Acylierung einer entsprechenden Diaminoverbindung oder durch Reduktion einer entsprechenden o-Nitro-acylaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid kann anschließend mittels Reduktion in eine entsprechende Verbindung der allgemeinen Formel I übergeführt werden.

Die anschließende Reduktion des erhaltenen N-Oxids der Formel I wird vorzugsweise in einem Lösungsmittel wie Wasser, Wasser/Ethanol, Methanol, Eisessig, Essigsäureethylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure,

Salzsäure oder Schwefelsäure, mit Salzen wie Eisen(II)sulfat, Zinn(II)chlorid oder Natriumdithionit, mit Derivaten des dreiwertigen Phosphors wie Triphenylphosphin, Triethylphosphit oder Phosphortrichlorid, oder mit Hydrazin in Gegenwart von Raney-Nickel bei Temperaturen zwischen 0 und 50 °C, vorzugsweise jedoch bei Raumtemperatur durchgeführt.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 °C, vorzugsweise bei Temperaturen zwischen 10 und 50 °C.

d) Zur Herstellung von Verbindungen der allgemeinen Formel I, in der B eine $-NR_3-CO$-Gruppe darstellt: Umsetzung einer Verbindung der allgemeinen Formel

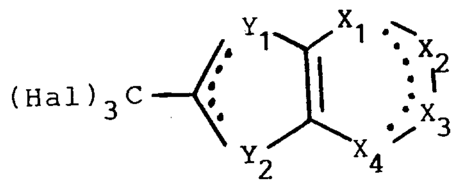

, (V)

in der

$X_1$ bis $X_4$, $Y_1$ und $Y_2$ wie eingangs definiert sind und Hal jeweils ein Chlor-, Brom- oder Jodatom darstellen, mit einer Verbindung der allgemeinen Formel

$A' - HNR_3$ ,(VI)

in der

$R_3$ wie eingangs definiert ist und

A' eine gegebenenfalls im Kohlenstoffgerüst durch eine oder zwei Alkylgruppen und am Stickstoffatom durch den Rest $R_a$ substituierte Piperidinylgruppe darstellt, in der $R_a$ mit der Maßgabe wie eingangs definiert ist, daß $R_a$ mit Ausnahme des Wasserstoffatoms wie eingangs definiert ist oder einen abspaltbaren Schutzrest für eine Iminogruppe wie die Acetyl-, Trifluoracetyl-, Benzoyl-, Benzyl-, Methoxybenzyl- oder 2,4-Dimethoxybenzylgruppe darstellt, und gegebenenfalls anschließende Abspaltung eines verwendeten Schutzrestes.

Die Umsetzung wird zweckmäßigerweise in einem Lösungsmittel wie Methanol/Wasser, Ethanol/Wasser, Isopropanol/Wasser oder Wasser gegebenenfalls in Gegenwart einer Base wie Natriumhydrogencarbonat bei Temperaturen zwischen 80 und 100 °C, vorzugsweise jedoch bei der Siedetemperatur des verwendeten Lösungsmittels, durchgeführt.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 °C, vorzugsweise bei Temperaturen zwischen 10 und 50 °C.

e) Zur Herstellung von Verbindungen der allgemeinen Formel I, in der F eine Alkoxycarbonylgruppe mit insgesamt 2 bis 7 Kohlenstoffatomen, eine Phenylalkoxycarbonylgruppe mit 1 bis 3 Kohlenstoffatomen im Alkoxyteil, eine Cycloalkoxycarbonyl- oder Cycloalkylalkoxycarbonylgruppe mit jeweils 3 bis 7 Kohlenstoffatomen im Cycloalkylteil und 1 bis 3 Kohlenstoffatomen im Alkoxyteil darstellt: Umsetzung einer Verbindung der allgemeinen Formel

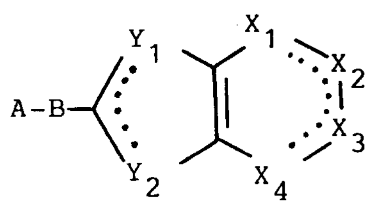

, (VII)

in der

A, B, $X_1$ bis $X_4$, $Y_1$ und $Y_2$ mit der Maßgabe wie eingangs definiert sind, daß F eine Carboxygruppe oder, falls $Z_3$ eine Hydroxygruppe darstellt, auch eine veresterte Carboxygruppe, z.B. eine Alkoxycarbonylgruppe mit insgesamt 2 bis 5 Kohlenstoffatomen oder eine Phenylalkoxycarbonylgruppe mit 1 bis 3 Kohlenstoffatomen im Alkoxyteil, bedeutet, mit einer Verbindung der allgemeinen Formel

$Z_3$ - $R_4$ ,(VIII)

in der

$R_4$ eine Alkylgruppe mit 1 bis 6 Kohlenstoffatomen, eine Phenylalkylgruppe mit 1 bis 3 Kohlenstoffatomen im Alkylteil, eine Cycloalkyl- oder Cycloalkylalkylgruppe mit jeweils 3 bis 7 Kohlenstoffatomen im Cycloalkylteil und 1 bis 3 Kohlenstoffatomen im Alkylteil und

$Z_3$ eine Hydroxygruppe oder, wenn F eine Carboxygruppe darstellt, eine nukleophile Austrittsgruppe wie ein Halogenatom oder eine Sulfonyloxygruppe, z.B. ein Chlor-, Brom- oder Jodatom, eine Methansulfonyloxy- oder p-Toluolsulfonyloxygruppe, darstellen.

Die Umsetzung wird zweckmäßigerweise in einem geeigneten Lösungsmittel, z. B. in einem entsprechenden Alkohol wie Methanol, Ethanol oder Isopropanol, Methylenchlorid, Tetrahydrofuran, Dioxan, Pyridin, Toluol oder Dimethylsulfoxid in Gegenwart eines säureaktivierenden und/oder wasserentziehenden Mittels wie Chlorwasserstoff, konz. Schwefelsäure, Thionylchlorid, Chlorameisensäureethylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, durch Umesterung, z. B. mit einem entsprechenden Kohlensäurediester, oder durch Umsetzung mit einem entsprechenden Halogenid vorzugsweise in Gegenwart einer Base wie Kaliumcarbonat und gegebenenfalls in Gegenwart eines Reaktionsbeschleunigers wie Kaliumjodid bei Temperaturen zwischen 0 und 100°C, vorzugsweise jedoch bei Temperaturen zwischen 20°C und der Siedetemperatur des betreffenden Lösungsmittels, durchgeführt.

Bedeutet $Z_3$ eine nukleophile Austrittsgruppe, so wird die Umsetzung vorzugsweise mit einem Alkalisalz einer Verbindung der allgemeinen Formel VII durchgeführt.

f) Zur Herstellung von Verbindungen der allgemeinen Formel I, in der A eine gegebenenfalls an einem Stickstoffatom durch eine Alkylgruppe mit 1 bis 3 Kohlenstoffatomen substituierte Amidinogruppe darstellt:

Umsetzung einer gegebenenfalls im Reaktionsgemisch gebildeten Verbindung der allgemeinen Formel

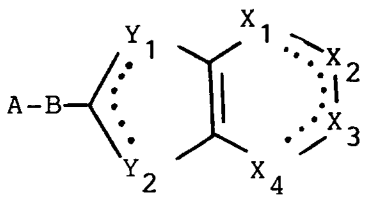

, (IX)

in der

A, B, $X_1$ bis $X_4$, $Y_1$ und $Y_2$ mit der Maßgabe wie eingangs definiert sind, daß A eine $Z_4$-C(=NH)-Gruppe darstellt, in der $Z_4$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_5$ - $NH_2$, ,(X)

in der

$R_5$ ein Wasserstoffatom oder eine Alkylgruppe mit 1 bis 3 Kohlenstoffatomen bedeutet, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise Ammoniumcarbonat oder Ammoniumacetat durchgeführt.

Eine Verbindung der allgemeinen Formel IX erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50°C, vorzugsweise jedoch bei Temperaturen zwischen 0 und 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

g) Zur Herstellung einer Verbindung der allgemeinen Formel I, in der A eine Amidino- oder Guanidinogruppe, in denen jeweils an einem der Stickstoffatome ein Wasserstoffatom durch eine Alkyl- oder Alkoxycarbonylgruppe, wobei die vorstehend erwähnten Alkyl- und Alkoxyteile jeweils 1 bis 3 Kohlenstoffatome enthalten können, ersetzt ist, oder eine gegebenenfalls im Kohlenstoffgerüst durch eine oder zwei Alkylgruppen und am Stickstoffatom durch den Rest $R_a$ substituierte Piperidinylgruppe, in der $R_a$ mit Ausnahme des Wasserstoffatoms wie eingangs definiert ist, wobei in der Piperidinylgruppe zusätzlich eine >CH-Einheit in 4-Stellung durch ein Stickstoffatom ersetzt sein kann:

Umsetzung einer Verbindung der allgemeinen Formel

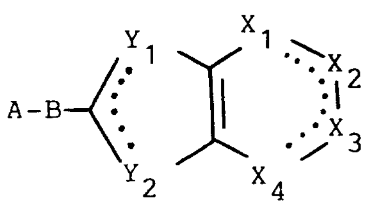

, (XI)

in der

A, B, $X_1$ bis $X_4$, $Y_1$ und $Y_2$ mit der Maßgabe wie eingangs definiert sind, daß A eine Amidino- oder Guanidinogruppe oder eine gegebenenfalls im Kohlenstoffgerüst durch eine oder zwei Alkylgruppen und am Stickstoffatom unsubstituierte Piperidinylgruppe, wobei in der Piperidinylgruppe zusätzlich eine >CH-Einheit in 4-Stellung durch ein Stickstoffatom ersetzt sein kann, darstellt, mit einer Verbindung der allgemeinen Formel

$Z_5$ - $R_6$ ,(XII)

in der

$R_6$ eine Alkoxycarbonylgruppe mit insgesamt 2 bis 5 Kohlenstoffatomen, eine Alkyl-, Phenylalkyl- oder Phenylalkoxycarbonylgruppe mit jeweils 1 bis 3 Kohlenstoffatome im Alkyl- oder Alkoxyteil oder eine $R_1$-CO-($R_2$CH)-O-CO-Gruppe, wobei $R_1$ und $R_2$ wie eingangs definiert sind, und

$Z_5$ eine nukleophile Austrittsgruppe wie ein Halogenatom, eine Aryloxy-, Arylthio-, Alkoxycarbonyloxy-, Aralkoxycarbonyloxy- oder N-Imidazolylgruppe, z. B. ein Chlor-, Brom- oder Jodatom oder eine 4-Nitrophenoxygruppe, oder, wenn $R_6$ eine Alkyl- oder Phenylalkylgruppe darstellt,

$Z_5$ zusammen mit dem Wasserstoffatom der benachbarten Methylengruppe des Restes $R_6$ auch ein Sauerstoffatom darstellen.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin, Pyridin oder 4-Dimethylamino-pyridin, welche gleichzeitig als Lösungsmittel dienen können, bei Temperaturen zwischen -30 und 100°C, vorzugsweise jedoch bei Temperaturen zwischen -10 und 60°C, durchgeführt.

Die Alkylierung mit einer Verbindung der Formel XII, in der $Z_5$ eine nukleophile Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100°C, vorzugsweise jedoch bei Temperaturen zwischen -10 und 80°C, durchgeführt.

Die reduktive Alkylierung mit einer Carbonylverbindung der allgemeinen Formel XII wird in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung wird jedoch vorzugsweise in Gegenwart von Ameisensäure als Reduktionsmittel bei erhöhten Temperaturen, z. B. bei Temperaturen zwischen 60 und 120 ° C, durchgeführt.

h) Zur Herstellung von Verbindungen der allgemeinen Formel I, in der D eine Sulfinyl- oder Sulfonylgruppe darstellt:

Oxidation einer Verbindung der allgemeinen Formel

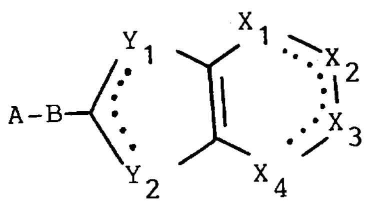

, (XIII)

in der

A, B, $X_1$ bis $X_4$, $Y_1$ und $Y_2$ mit der Maßgabe wie eingangs definiert sind, daß D ein Schwefelatom oder eine Sulfinylgruppe darstellt.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100 ° C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20 ° C oder in Aceton bei 0 bis 60 ° C, mit einer Persäure wie Perameisensäure in Eisessig oder Trifluoressigsäure bei 0 bis 50 ° C oder mit m-Chlorperbenzoesäure in Methylenchlorid oder Chloroform bei -20 bis 60 ° C, mit Natriummetaperjodat in wäßrigem Methanol oder Ethanol bei -15 bis 25 ° C, mit Brom in Eisessig oder wäßriger Essigsäure gegebenenfalls in Gegenwart einer schwachen Base wie Natriumacetat, mit N-Brom-succinimid in Ethanol, mit tert.Butyl-hypochlorit in Methanol bei -80 bis -30 ° C, mit Jodbenzodichlorid in wäßrigem Pyridin bei 0 bis 50 ° C, mit Salpetersäure in Eisessig bei 0 bis 20 ° C, mit Chromsäure in Eisessig oder in Aceton bei 0 bis 20 ° C und mit Sulfurylchlorid in Methylenchlorid bei -70 ° C, der hierbei erhaltene Thioether-Chlor-Komplex wird zweckmäßigerweise mit wäßrigem Ethanol hydrolysiert.

Zur Herstellung einer S,S-Dioxidverbindung der allgemeinen Formel I wird die Oxidation ausgehend von einer entsprechenden Alkylsulfinylverbindung zweckmäßigerweise mit einem oder mehr Äquivalenten des verwendeten Oxidationsmittels oder ausgehend von einer entsprechenden Alkylsulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100 ° C oder in Aceton bei 0 bis 60 ° C, mit einer Persäure wie Perameisensäure oder m-Chlorperbenzoesäure in Eisessig, Trifluoressigsäure, Methylenchlorid oder Chloroform bei Temperaturen zwischen 0 und 60 ° C, mit Salpetersäure in Eisessig bei 0 bis 20 ° C, mit Chromsäure oder Kaliumpermanganat in Eisessig, Wasser/Schwefelsäure oder in Aceton bei 0 bis 20 ° C.

i) Zur Herstellung von Verbindungen der allgemeinen Formel I, in der einer der Reste $X_1$ bis $X_4$ eine F-E-D-N$<$ Gruppe darstellt, wobei D eine geradkettige oder verzweigte Alkylgruppe mit 1 bis 6 Kohlenstoffatomen oder eine Alkenylgruppe mit 2 bis 6 Kohlenstoffatomen darstellt:

Umsetzung einer Verbindung der allgemeinen Formel

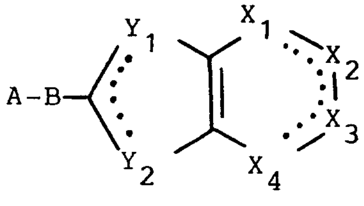

, (XIV)

in der
A, B, $Y_1$, $Y_2$ und $X_1$ bis $X_4$ mit der Maßgabe wie eingangs definiert sind, daß einer der Reste $X_1$ bis $X_4$ eine H-N< Gruppe darstellt, mit einer Verbindung der allgemeinen Formel

$Z_6$ - D' - E - F ,(XV)

in der
E und F wie eingangs definiert sind,
D' eine geradkettige oder verzweigte Alkylgruppe mit 1 bis 6 Kohlenstoffatomen oder eine Alkenylgruppe mit 2 bis 6 Kohlenstoffatomen und
$Z_6$ eine Austrittsgruppe, z.B. ein Chlor-, Brom- oder Jodatom, eine Hydroxy-, Methoxy-, Ethoxy- oder Benzyloxygruppe, oder, falls D' eine unmittelbar an den Rest F gebundene Kohlenstoff-Kohlenstoff-Doppelbindung enthält, auch ein Wasserstoffatom bedeuten.

Die Umsetzung wird zweckmäßigerweise in einem geeigneten Lösungsmittel, z. B. in einem entsprechenden Alkohol wie Methanol, Ethanol oder Isopropanol, in Methylenchlorid, Tetrahydrofuran, Dioxan, Pyridin, Toluol oder Dimethylsulfoxid, gegebenenfalls in Gegenwart einer Base wie Kaliumcarbonat oder Natriumhydrid und gegebenenfalls in Gegenwart eines Reaktionsbeschleunigers wie Kaliumjodid bei Temperaturen zwischen 0 und 100 °C, vorzugsweise jedoch bei Temperaturen zwischen 20 °C und der Siedetemperatur des betreffenden Lösungsmittels, durchgeführt.

Bedeutet $Z_6$ eine Hydroxygruppe, so wird die Umsetzung vozugsweise in Gegenwart eines aktivierenden Mittels wie Triphenylphosphin/Azodicarbon-säure-diethylester durchgeführt.

j) Zur Herstellung von Verbindungen der allgemeinen Formel I, in der D eine Alkylengruppe mit 2 bis 6 Kohlenstoffatomen darstellt:
Katalytische Hydrierung einer Verbindung der allgemeinen Formel

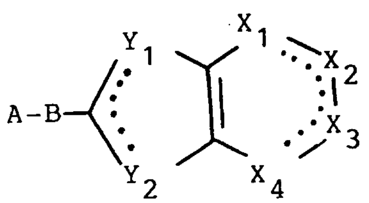

, (XVI)

in der
A, B, $Y_1$, $Y_2$ und $X_1$ bis $X_4$ mit der Maßgabe wie eingangs definiert sind, daß D eine Alkenylengruppe mit 2 bis 6 Kohlenstoffatomen darstellt.

Die katalytische Hydrierung wird in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50 °C, z.B. bei Raumtemperatur, und einem Wasserstoffdruck von 1 bis 10 bar durchgeführt. Bei der katalytischen Hydrierung können gleichzeitig andere Reste, z.B. eine Nitrogruppe zur Aminogruppe oder eine Benzyloxygruppe zur Hydroxygruppe und eine Benzylamino- oder Benzyloxycarbonylaminogruppe zu einer Aminogruppe mitreduziert werden. Außerdem können gleichzeitig andere C = C-Doppelbindungen zu Einfachbindungen aufhydriert werden.

k) Zur Herstellung von Verbindungen der allgemeinen Formel I, in der A eine durch eine Hydroxygruppe substituierte Amidinogruppe darstellt:
Umsetzung einer Verbindung der allgemeinen Formel

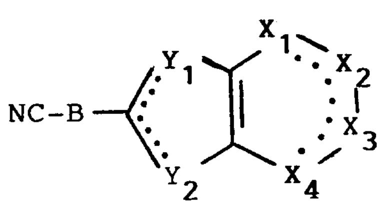

, (XVII)

in der
B, $Y_1$, $Y_2$, und $X_1$ bis $X_4$ wie eingangs definiert sind, mit Hydroxylamin oder dessen Salzen in Gegenwart

einer Base.

Die Umsetzung wird vorzugsweise in einem Lösungsmittel wie Methanol, Wasser/Methanol, Ethanol, Ethanol/Wasser oder Tetrahydrofuran/Wasser gegebenenfalls in Gegenwart einer Base wie Natriumcarbonat, Kaliumcarbonat oder N,N-Diisopropyl-ethylamin bei Temperaturen zwischen 20 und 100 °C, vorzugsweise jedoch bei der Siedetemperatur des verwendeten Lösungsmittels, durchgeführt.

l) Zur Herstellung von Verbindungen der allgemeinen Formel I, in der mindestens einer der Reste $X_1$ bis $X_4$ eine Methingruppe darstellt:

Enthalogenierung einer Verbindung der allgemeinen Formel

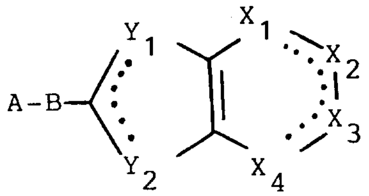

, (XVIII)

in der

A, B, $Y_1$, $Y_2$ und $X_1$ bis $X_4$ mit der Maßgabe wie eingangs definiert sind, daß mindestens einer der Reste $X_1$ bis $X_4$ eine durch ein Chlor-, Brom- oder Jodatom substituierte Methingruppe darstellt.

Die Enthalogenierung wird in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50 °C, z.B. bei Raumtemperatur, und einem Wasserstoffdruck von 1 bis 10 bar durchgeführt. Bei der katalytischen Hydrierung können gleichzeitig andere Reste, z.B. eine Nitrogruppe zur Aminogruppe oder eine Benzyloxygruppe zur Hydroxygruppe und eine Benzylamino- oder Benzyloxycarbonylaminogruppe zu einer Aminogruppe mitreduziert werden. Außerdem können gleichzeitig andere C = C-Doppelbindungen zu Einfachbindungen aufhydriert werden.

Erhält man erfindungsgemäß eine Verbindung der allgemeinen Formel I, in der mindestens einer der Reste $X_1$ bis $X_4$ eine Carbonylgruppe darstellt, so kann diese mittels Halogenierung in eine entsprechende Verbindung der allgemeinen Formel I, in der mindestens einer der Reste $X_1$ bis $X_4$ eine durch ein Chlor- oder Bromatom substituierte Methingruppe darstellt, übergeführt werden.

Die nachträgliche Halogenierung wird vorzugsweise mit einem Halogenierungsmittel wie Phosphoroxychlorid, Phosphoroxybromid oder Phosphorpentachlorid in einem inerten Lösungsmittel wie Toluol, wobei jedoch vorzugsweise als Lösungsmittel ein Überschuß des eingesetzten Halogenierungsmittels wie Phosphoroxychlorid verwendet wird, bei erhöhten Tempteraturen, z.B. bei Temperaturen zwischen 80 und 120 °C, durchgeführt.

Bei den vorstehend beschriebenen Umsetzungen können gegebenenfalls vorhandene reaktive Gruppen wie Hydroxy-, Carboxy-, Phosphono-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe,

als Schutzrest für eine Phosphonogruppe die Trimethylsilyl-, Methyl-, Ethyl- oder Benzylgruppe und

als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser,Tetrahydrofuran/Wasser oder Dioxan/Wasser, in Gegenwart einer Säure wie Trifluoressigsäure, Salzsäure, Bromwasserstoffsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 °C, vorzugsweise bei Temperaturen zwischen 10 und 50 °C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C vorzugsweise jedoch bei Raumtemperatur,

und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer-(IV)ammoniumnitrat in einem Lösungsmittel wie Methylenchlorid, Acetonitril oder Acetonitril/Wasser bei Temperaturen zwischen 0 und 50 °C, vorzugsweise jedoch bei Raumtemperatur, erfolgen.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei Temperaturen zwischen 40 und 150 °C, vorzugsweise jedoch bei Temperaturen zwischen 60 und 100 °C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen 0 °C und der Siedetemperatur des Reaktionsgemisches, vorzugsweise jedoch bei Temperaturen zwischen 20 und 60 °C.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 °C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese in den Beispielen I bis XV beschrieben werden.

Wie bereits eingangs erwähnt, weisen die neuen kondensierten 5-gliedrigen Heterocyclen der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I, in denen A eine gegebenenfalls an einem der Stickstoffatome durch eine Alkylgruppe oder eine in vivo abspaltbare Gruppe substituierte Amidino- oder Guanidinogruppe oder eine gegebenenfalls im Kohlenstoffgerüst durch eine oder zwei Alkylgruppen und am Stickstoffatom durch den gegebenenfalls in vivo abspaltbaren Rest $R_a$ substituierte Piperidinylgruppe, in der zusätzlich eine >CH- Einheit in 4-Stellung durch ein Stickstoffatom ersetzt sein kann, und F eine Carboxy-, Sulfo-, Phosphono-, O-Alkyl-phosphono- oder 5-Tetrazolylgruppe oder eine in vivo in eine Carboxy-, Sulfo-, Phosphono- oder O-Alkylphosphonogruppe überführbare Gruppe darstellen, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in der F eine O,O-Dialkyl-phosphonogruppe darstellt, stellen wertvolle Zwischenprodukte zur Herstellung der entsprechenden Verbindungen der allgemeinen Formel I dar.

Beispielsweise wurden die Verbindungen der allgemeinen Formel I auf ihre biologischen Wirkungen wie folgt untersucht:

1. Hemmung der Bindung von $^3$H-BIBU 52 an Humanthrombozyten:

Eine Suspension von Humanthrombozyten in Plasma wird mit $^3$H-BIBU 52 [= (3S,5S)-5-[(4'-Amidino-4-biphenylyl)oxymethyl]-3-[(carboxyl)methyl]-2-pyrrolidinon[3-$^3$H-4-biphenylyl]], das den literaturbekannten Liganden $^{125}$J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 ml werden mit 50 ml physiologischer Kochsalzlösung, 100 ml Testsubstanzlösung, 50 ml $^{14}$C- Sucrose (3.700 Bq) und 50 ml $^3$H-BIBU 52 (Endkonzentration: 5 nM) bei Raumtemperatur 20 Minuten inkubiert. Zur Messung der unspezifischen Bindung wird anstelle der Testsubstanz 5 ml BIBU 52 (Endkonzentration: 30 mM) eingesetzt.
Die Proben werden 20 Sekunden bei 10000 x g zentrifugiert und der Überstand abgezogen. 100 ml hiervon werden zur Bestimmung des freien Liganden gemessen. Das Pellet wird in 500 ml 0,2N NaOH gelöst, 450 ml werden mit 2 ml Szintillator und 25 ml 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung:

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$ [nM] | Hemmung der Plättchenaggregation $EC_{50}$ [nM] |
|---|---|---|
| 1 | 110 | 430 |
| 1(1) | 26000 | >10000 |
| 2 | 200 | 2000 |
| 1(15) | 490 | 1250 |
| 1(48) | 280 | 250 |
| 1(49) | 270 | 150 |
| 1(61) | 150 | 110 |
| 1(63) | 9800 | 4400 |
| 1(64) | 50000 | 30000 |
| 1(67) | 1100 | 2300 |
| 6(12) | 24000 | 6400 |
| 7(12) | 1300 | 660 |

Die erfindungsgemäßen Verbindungen sind gut verträglich, da beispielsweise bei intravenöser Gabe von 30 mg/kg an jeweils drei Mäusen bei der Verbindung des Beispiels 1 kein Tier gestorben war.

Auf Grund ihrer Hemmwirkung auf Zell-Zell- bzw. Zell-Matrix-Wechselwirkungen eignen sich die neuen kondensierten 5-gliedrigen Heterocyclen der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z. B. bei der Bekämpfung bzw. Verhütung von venösen und arteriellen Thrombosen, von zerebrovasculären Erkrankungen, von Lungenembolien, des Herzinfarkts, der Arteriosklerose, der Osteoporose und der Metastasierung von Tumoren und der Therapie genetisch bedingter oder auch erworbener Störungen der Interaktion von Zellen untereinander oder mit soliden Strukturen. Weiterhin eignen sich diese bei der Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 mg und 30 mg/kg Körpergewicht, vorzugsweise bei 1 mg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, $\alpha$-Rezeptorantagonisten, Alkylnitrate wie Glycerintrinitrat, Phosphodiesterasehemmer, Prostacyclin und dessen Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

## Beispiel I

3-(1-Benzyloxycarbonyl-4-piperidinyl)-propionylchlorid

3,09 g 3-(1-Benzyloxycarbonyl-4-piperidinyl)-propionsäure, 50 ml Methylenchlorid und 2,08 g Phosphorpentachlorid werden zusammengegeben und 6 Stunden bei Raumtemperatur stehen gelassen. Man dampft im Vakuum ein und dampft mit Toluol nach. Der verbleibende Rückstand wird ohne weitere Reinigung weiterverwendet.
Ausbeute: 3,1 g (100 % der Theorie)

Beispiel II

3-(1-Benzyloxycarbonyl-4-piperidinyl)-propionsäure

24,6 g 3-(4-Piperidinyl)-propionsäure werden in 170 ml 1N Natronlauge gelöst, auf 0 °C abgekühlt und unter Einsatz eines Vibromischers innerhalb von 30 Minuten tropfenweise mit 175 ml 1N Natronlauge und 25 ml Benzyloxycarbonylchlorid, die beide getrennt zugegeben werden, versetzt. Man läßt weitere 60 Minuten bei 0 °C nachreagieren und dann 16 Stunden bei Raumtemperatur stehen. Man wäscht mit 125 ml eines 4:1-Gemisches aus Ether/Petrolether, säuert die wäßrige Phase mit 4N Salzsäure an und extrahiert mit Ether. Die Etherextrakte werden im Vakuum eingedampft. Der ölige Rückstand wird direkt weiter verwendet.
Ausbeute: 50 g (100 % der Theorie),
$R_f$-Wert: 0,65 (Kieselgel; Methylenchlorid/Methanol/Eisessig = 5:1:0,1)
Analog wird folgende Verbindung erhalten:
(1) trans-4-(Benzyloxycarbonylamino-methyl)-cyclohexancarbonsäure
Schmelzpunkt: 109-110 °C

Beispiel III

5-[(2-Methoxycarbonyl-ethyl)-aminocarbonyl]-1-methyl-2-trichlormethyl-benzimidazol

10 g 3-Amino-4-methylamino-benzoesäure-[N-(2-methoxycarbonyl-ethyl)-amid] werden in 35 ml Eisessig gelöst und innerhalb von 20 Minuten tropfenweise mit einer Lösung von 7,05 g Trichloracetimidsäuremethylester in 5 ml Eisessig versetzt.Man rührt noch 16 Stunden bei Raumtemperatur, destilliert den Eisessig im Vakuum ab und verwendet den Rückstand ohne weitere Reinigung.
Ausbeute: 15 g (100 % der Theorie),
$R_f$-Wert: 0,71 (Kieselgel; Essigester/Ethanol = 7:3)

Beispiel IV

5-(3-Amino-4-methylamino-phenyl)-5-oxo-valeriansäure-isopropylester

2,4 g 5-(4-Methylamino-3-nitro-phenyl)-5-oxo-valeriansäureisopropylester werden in 80 ml Isopropanol in Gegenwart von 0,1 g 10%iger Palladiumkohle (weitere 0,1 g werden nach 40 Stunden zugegeben) 48 Stunden mit Wasserstoff von 4 bar bei Raumtemperatur hydriert. Man filtriert den Katalysator ab, dampft im Vakuum ein und reinigt den Rückstand über Kieselgel (Elutionsmittel: Essigester/Petrolether = 8:2).
Ausbeute: 1,0 g (46 % der Theorie),
$R_f$-Wert: 0,67 (Kieselgel; Essigester/Ethanol = 9:1)

Beispiel V

4-Amino-1-(4-ethoxycarbonyl-butyl)-3-methyl-1H,3H-pyrimidin-2,6-dion

Hergestellt aus 4-Amino-3-methyl-1H,3H-pyrimidin-2,6-dion analog Beispiel 11(4) mit festem Natriumhydroxid als Base.
Schmelzpunkt: 110-112 °C
Analog werden folgende Verbindungen erhalten:
(1) 5-(4-Cyano-benzoylamino)-1-(4-ethoxycarbonyl-butyl)-3-(4-methoxy-benzyl)-4-methylamino-1H,3H-pyrimidin-2,6-dion Man arbeitet in Dimethylformamid mit Kalium-tert.butylat.
$R_f$-Wert: 0,52 (Kieselgel; Methylenchlorid/Methanol/konz. Ammoniak = 9:1:0,1)
(2) 9-(4-Cyano-benzoylamino)-7-(4-ethoxycarbonyl-butyl)-3,4-dihydro-2H,7H-pyrimido[1,6-a]pyrimidin-6,8-dion
$R_f$-Wert: 0,55 (Kieselgel; Methylenchlorid/Methanol/Eisessig = 19:1:0,1)

Beispiel VI

8-(4-Cyano-phenyl)-3,9-dimethyl-xanthin

2,1 g 5-Amino-1-methyl-6-methylamino-1H,3H-pyrimidin-2,4-dion werden in 150 ml Tetrahydrofuran suspendiert und unter Rühren und Rückflußkochen 9,1 g 4-Dimethylamino-pyridin zugegeben. Nach 15 Minuten fügt man 2,2 g 4-Cyano-benzoylchlorid zu und erhitzt unter Rühren weitere 2 Stunden zum Rückfluß. Die Reaktionsmischung wird eingedampft und der Rückstand 45 Minuten auf 200 bis 205 °C erhitzt. Nach dem Abkühlen wird das Produkt mit 15 ml Methanol verrieben, abfiltriert und mit Methanol und Ether gewaschen und in dieser Form weiterverwendet.
Ausbeute: 2,0 g (58 % der Theorie),
$R_f$-Wert: 0,79 (Kieselgel; Methylenchlorid/Methanol/Eisessig = 9:1:0,1, nach zweimaliger Entwicklung)
Analog werden folgende Verbindungen erhalten:

(1) 8-[2-(1-Benzyloxycarbonyl-4-piperidinyl)-ethyl]-3,9-dimethyl-xanthin
a) 5-[[2-(1-Benzyloxycarbonyl-4-piperidinyl)-ethyl]-carbonylamino]-1-methyl-6-methylamino-uracil
Man erhitzt 5 Stunden unter Rückfluß und digeriert den nach dem Eindampfen des Tetrahydrofurans verbliebenen Rückstand mit Essigester/Ethanol (3:1)
$R_f$-Wert: 0,79 (Kieselgel; Methylenchlorid/Methanol/konz. Ammoniak = 7:3:0,1)
b) 8-[2-(1-Benzyloxycarbonyl-4-piperidinyl)-ethyl]-3,9-dimethyl-xanthin
Man erhitzt 10 Minuten auf 250 °C
$R_f$-Wert: 0,62 (Kieselgel; Methylenchlorid/Methanol/Eisessig = 8:2:0,1)

(2) 2-[2-(1-Benzyloxycarbonyl-4-piperidinyl)-ethyl]-8,10-dioxo-5,6-dihydro-4H,9H-pyrimido[1,2,3-cd]purin
a) 9-[[2-(1-Benzyloxycarbonyl-4-piperidinyl)-ethyl]-carbonylamino]-3,4-dihydro-2H,7H-pyrimido[1,6-a]-pyrimidin-6,8-dion Man verwendet 3-(1-Benzyloxycarbonyl-4-piperidinyl)-propionsäure und Carbonyldiimidazol in Dimethylformamid. $R_f$-Wert: 0,73 (Kieselgel; Methylenchlorid/Methanol = 8:2)
b) 2-[2-(1-Benzyloxycarbonyl-4-piperidinyl)-ethyl]-8,10-dioxo-5,6-dihydro-4H,9H-pyrimido[1,2,3-cd]-purin
Man erwärmt 1,5 Stunden auf 240 °C
$R_f$-Wert: 0,52 (Kieselgel; Methylenchlorid/Methanol = 9:1)

(3) 8-[2-(1-Benzyloxycarbonyl-4-piperidinyl)-ethyl]-9-isopropyl-xanthin
a) 5-[[2-(1-Benzyloxycarbonyl-4-piperidinyl)-ethyl]-carbonylamino]-4-isopropylamino-1H,3H-pyrimidin-2,6-dion Man verfährt analog (2a).
$R_f$-Wert: 0,59 (Kieselgel; Methylenchlorid/Methanol/Eisessig = 19:1:0,1, nach dreimaliger Entwicklung)
b) 8-[2-(1-Benzyloxycarbonyl-4-piperidinyl)-ethyl]-9-isopropyl-xanthin
Man arbeitet in Portionen von nicht mehr als 2,5 g und erhitzt maximal 10 Minuten auf 250-275 °C
$R_f$-Wert: 0,69 (Kieselgel; Methylenchlorid/Methanol/Eisessig = 19:1:0,1, nach dreimaliger Entwicklung)

(4) 8-(4-Cyano-phenyl)-3,7-dimethyl-xanthin
a) 4-Amino-5-[N-(4-cyanobenzoyl)-N-methyl-amino]-3-methyl-1H,3H-pyrimidin-2,6-dion
Man arbeitet in Tetrahydrofuran und Natronlauge bei Raumtemperatur. Das Ausgangsmaterial 4-Amino-3-methyl-5-methylamino-1H,3H-pyrimidin-2,6-dion erhält man aus 4-Amino-5-brom-3-methyl-1H,3H-pyrimidin-2,6-dion.
Schmelzpunkt: über 275 °C
$R_f$-Wert: 0,29 (Kieselgel; Methylenchlorid/Methanol/Eisessig = 9:1:0,1)
b) 8-(4-Cyano-phenyl)-3,7-dimethyl-xanthin
Man erhitzt 10 Minuten auf 250 °C in Gegenwart von 4-Dimethylamino-pyridin
Schmelzpunkt: über 300 °C
$R_f$-Wert: 0,30 (Kieselgel; Methylenchlorid/Methanol/Eisessig = 9:1:0,1)

(5) 8-[2-(1-Benzyloxycarbonyl-4-piperidinyl)-ethyl]-3,7-dimethyl-xanthin
a) 4-Amino-5-[[2-(1-benzyloxycarbonyl-4-piperidinyl)-ethyl]-carbonylamino]-3-methyl-1H,3H-pyrimidin-2,6-dion
Man arbeitet analog (2a), jedoch mit O-(1H-1-Benztriazolyl)-N,N,N',N'-tetramethyluronium-tetrafluorborat in Gegenwart von Triethylamin.
Schmelzpunkt: 223-224 °C (sintert ab 220 °C)
$R_f$-Wert: 0,34 (Kieselgel; Methylenchlorid/Methanol/Eisessig = 15:1:0,1, nach zweimaliger Entwicklung)

b) 8-[2-(1-Benzyloxycarbonyl-4-piperidinyl)-ethyl]-3,7-dimethyl-xanthin.
Man erhitzt 10 Minuten auf 275°C
Schmelzpunkt: 200-203°C (sintert ab 198°C)
$R_f$-Wert: 0,88 (Kieselgel; Methylenchlorid/Methanol/Eisessig = 15:1:0,1, nach zweimaliger Entwicklung)

(6) 8-[4-(1-Benzyloxycarbonyl-4-piperidinyl)-phenyl]-3,9-dimethyl-xanthin

a) 5-[4-(1-Benzyloxycarbonyl-4-piperidinyl)-benzoylamino]-3-methyl-4-methylamino-1H,3H-pyrimidin-2,6-dion
Man verfährt analog (5a).
Schmelzpunkt: 262-265°C (Zers.)
$R_f$-Wert: 0,35 (Kieselgel; Methylenchlorid/Methanol/konz. Ammoniak = 9:1:0,1, nach zweimaliger Entwicklung)

b) 8-[4-(1-Benzyloxycarbonyl-4-piperidinyl)-phenyl]-3,9-dimethyl-xanthin.
Man erhitzt trocken auf 259°C, fügt unter Rühren Diphenylether zu und hält insgesamt 3 Stunden bei dieser Temperatur. Schmelzpunkt: 260-263°C.
$R_f$-Wert: 0,50 (Kieselgel; Methylenchlorid/Methanol/Eisessig = 9:1:0,1)

(7) 5-(4-Cyano-benzoylamino)-3-(4-methoxy-benzyl)-4-methylamino-1H,3H-pyrimidin-2,6-dion
Man verfährt analog (1a).
Schmelzpunkt: 245-250°C (Zers.)
$R_f$-Wert: 0,40 (Kieselgel; Methylenchlorid/Methanol/konz. Ammoniak = 9:1:0,1)

(8) 9-(4-Cyano-benzoylamino)-3,4-dihydro-2H,7H-pyrimido-[1,6-a]pyrimidin-6,8-dion
Man verfährt analog (1a).
Schmelzpunkt: über 290°C
$R_f$-Wert: 0,32 (Kieselgel; Methylenchlorid/Methanol/Eisessig = 9:1:0,1)

(9) 3-[4-(1-tert.Butyloxycarbonyl-4-piperidinyl)-benzoylamino]-4-methylamino-zimtsäure-methylester
Man arbeitet analog (5a)
$R_f$-Wert: 0,64 (Kieselgel;Essigester/Petrolether = 7:3)

Beispiel VII

5-(4-Methylamino-3-nitro-phenyl)-5-oxo-valeriansäure-isopropylester

a) 5-(4-Methylamino-3-nitro-phenyl)-5-oxo-valeriansäure
13,6 g 5-(4-Chlor-3-nitro-phenyl)-5-oxo-valeriansäure (hergestellt durch Nitrierung von 5-(4-Chlor-phenyl)-5-oxo-valeriansäure mit rauchender Salpetersäsure bei -5°C) werden mit 250 ml 40%iger wäßriger Methylaminlösung in einem Druckgefäß 8 Stunden auf 50°C erhitzt. Die Reaktionslösung wird im Vakuum eingedampft, der Niederschlag in Wasser aufgenommen und mit Salzsäure angesäuert. Das ausgefallen Produkt wird ohne weitere Reinigung weiterverwendet. $R_f$-Wert: 0,35 (Kieselgel; Essigester/Ethanol = 9:1)

b) 5-(4-Methylamino-3-nitro-phenyl)-5-oxo-valeriansäureisopropylester
Die erhaltene rohe 5-(4-Methylamino-3-nitro-phenyl)-5-oxo-valeriansäure wird mit 300 ml isopropanolischer Salzsäure 6 Stunden auf dem Dampfbad erhitzt. Man dampft im Vakuum ein und reinigt den Rückstand durch zweimalige Chromatographie an Kieselgel (Elutionsmittel: erst Essigester/Ethanol = 100:1, dann Essigester/Petrolether = 1:1).
Ausbeute: 2,4 g (15 % der Theorie),
$R_f$-Wert: 0,80 (Kieselgel; Essigester/Ethanol = 9:1)

Analog werden folgende Verbindungen erhalten:

(1) 3-Amino-4-methylamino-zimtsäure-methylester
Man verfährt analog b).
$R_f$-Wert: 0,65 (Kieselgel; Essigester/Petrolether = 7:3)

(2) 4-Methylamino-3-nitro-zimtsäure
Hergestellt analog a). Das Ausgangsmaterial erhält man durch Erhitzen von 4-Chlor-3-nitro-benzaldehyd in Acetanhydrid in Gegenwart von Natriumacetat.
$R_f$-Wert: 0,83 (Methylenchlorid/Methanol = 50:1)

Beispiel IX

9-Amino-3,4-dihydro-2H,7H-pyrimido[1,6-a]pyrimidin-6,8-dion

3,4 g 9-Nitroso-3,4-dihydro-2H,7H-pyrimido[1,6-a]pyrimidin-6,8-dion werden in 250 ml Methanol in Gegenwart von 2,5 g Raney-Nickel bei Raumtemperatur 16 Stunden mit Wasserstoff von 5 bar behandelt. Man fügt 25 ml Wasser zu, erhitzt auf dem Dampfbad und filtriert heiß. Das Filtrat wird eingedampft und der Rückstand roh weiter verarbeitet.
Ausbeute: 3 g (95% der Theorie),
$R_f$-Wert: 0,13 (Kieselgel; Essigester/Ethanol = 7:3)
Analog werden folgende Verbindungen erhalten:
(1) 5-Amino-4-isopropylamino-1H,3H-pyrimidin-2,6-dion
$R_f$-Wert: 0,52 (Kieselgel; Methylenchlorid/Methanol/Eisessig = 8:2:0,1)
(2) 4,5-Diamino-1-(4-ethoxycarbonyl-butyl)-3-methyl-1H,3H-pyrimidin-2,6-dion
$R_f$-Wert: 0,84 (Kieselgel; Methylenchlorid/Methanol/konz. Ammoniak = 9:1:0,1, nach zweimaliger Entwicklung)
(3) 5-Amino-3-(4-methoxy-benzyl)-4-methylamino-1H,3H-pyrimidin-2,6-dion
$R_f$-Wert: 0,50 (Kieselgel; Essigester/Ethanol/konz. Ammoniak = 8:2:0,1)

Beispiel X

9-Nitroso-3,4-dihydro-2H,7H-pyrimido[1,6-a]pyrimidin-6,8-dion

3,35 g 3,4-Dihydro-2H,7H-pyrimido[1,6-a]pyrimidin-6,8-dion werden auf dem Dampfbad in 65 ml Wasser gelöst und nach Zufügen von 1,63 g Natriumnitrit im Laufe von 30 Minuten tropfenweise mit 2,75 ml Eisessig versetzt. Man rührt 30 Minuten nach, engt auf die Hälfte des Volumens ein, kühlt mit Eiswasser und filtriert den Niederschlag ab.
Ausbeute: 3,5 g (90% der Theorie)
$R_f$-Wert: 0,02 (Kieselgel; Essigester/Ethanol = 7:3)
Analog werden folgende Verbindungen erhalten:
(1) 4-Isopropylamino-5-nitroso-1H,3H-pyrimidin-2,6-dion
Schmelzpunkt: 224-226 °C (Zers.)
(2) 4-Amino-1-(4-ethoxycarbonyl-butyl)-3-methyl-5-nitroso-1H,3H-pyrimidin-2,6-dion.
Man arbeitet bei Raumtemperatur.
Schmelzpunkt: 182-183 °C (Zers.)
(3) 3-(4-Methoxy-benzyl)-4-methylamino-5-nitroso-1H,3H-pyrimidin-2,6-dion.
Schmelzpunkt: 167 °C (Zers.)

Beispiel XI

3,4-Dihydro-2H,7H-pyrimido[1,6-a]pyrimidin-6,8-dion

16 g 6-[(3-Toluolsulfonyloxy-propyl)-amino]-1H,3H-pyrimidin-4,6-dion (hergestellt aus 6-Chlor-uracil und 3-Amino-propanol und anschließende Umsetzung mit Toluolsulfonylchlorid) werden in 100 ml 1N Natronlauge 2 Stunden auf 90 °C erhitzt. Man puffert mit Ammonchlorid ab und digeriert das ausgefallene Produkt mit Essigester.
Ausbeute: 3,5 g (45% der Theorie),
$R_f$-Wert: 0,89 (Reversed Phase Platte RP18; Methanol/5%-ige Natriumchloridlösung = 6:4)

Beispiel XII

5-(4-Cyano-benzoylamino)-1-(4-ethoxycarbonyl-butyl)-4-methylamino-1H,3H-pyrimidin-2,6-dion

3,3 g 5-(4-Cyano-benzoylamino)-1-(4-ethoxycarbonyl-butyl)-3-(4-methoxy-benzyl)-4-methylamino-1H,3H-pyrimidin-2,6-dion werden in 30 ml Trifluoressigsäure 4 Tage unter Stickstoff bei 65 °C gerührt. Man dampft ein, verreibt den Rückstand mit Wasser und filtriert das erhaltene Festprodukt ab.
Ausbeute: 1,62 g (63% der Theorie),
Schmelzpunkt: über 200 °C

$R_f$-Wert: 0,33 (Kieselgel; Methylenchlorid/Methanol = 9:1)

Beispiel XIII

3-(4-Methoxy-benzyl)-4-methylamino-1H,3H-pyrimidin-2,6-dion

68 g 4-Chlor-3-(4-methoxy-benzyl)-1H,3H-pyrimidin-2,6-dion werden mit 150 ml 40%iger wäßriger Methylaminlösung in der Bombe eine Stunde auf 120 °C erhitzt. Man versetzt mit 500 ml Wasser, filtriert das Festprodukt ab und kocht es mit Ethanol aus.
Ausbeute: 17 g (22% der Theorie),
$R_f$-Wert: 0,55 (Kieselgel; Essigester/Ethanol = 8:2)

Beispiel XIV

4-Chlor-3-(4-methoxy-benzyl)-1H,3H-pyrimidin-2,6-dion

Eine Mischung aus 45,1 g 6-Chlor-uracil, 30,9 g Kalimhydrogencarbonat und 300 ml Dimethylsulfoxid wird 2 Stunden auf 80 °C erwärmt. Man fügt 41,9 g 4-Methoxy-benzylchlorid zu und erwärmt weitere 4 Stunden auf 80 °C. Das Lösungsmittel wird im Vakuum abgedampft, der Rückstand auf 2000 ml Wasser gegossen und das erhaltene Festprodukt abfiltriert.
Ausbeute: 68 g (96% der Theorie),
Schmelzpunkt: 270-274 °C
$R_f$-Wert: 0,90 (Kieselgel; Essigester/Ethanol = 7:3)

Beispiel XV

3-Amino-4-methylamino-zimtsäure

11 g 4-Methylamino-3-nitro-zimtsäure werden in 190 ml siedender 5%iger Natronlauge solange mit Natriumdithionit versetzt, bis die rote Farbe verschwunden ist (insgesamt 31 g). Das Produkt kristallisiert beim Abkühlen aus.
Ausbeute: 6 g (63% der Theorie),
$R_f$-Wert: 0,31 (Kieselgel; Essigester/Ethanol = 7:3)

Beispiel 1

5-[(2-Carboxy-ethyl)-aminocarbonyl]-1-methyl-2-[2-(4-piperidinyl)-ethyl]-benzimidazol-dihydrobromid

0,4 g 5-[(2-Methoxycarbonyl-ethyl)-aminocarbonyl]-1-methyl-2-[2-(4-piperidinyl)-ethyl]-benzimidazol werden mit 2 ml 48%iger Bromwasserstoffsäure 18 Stunden bei Raumtemperatur gerührt. Man dampft im Vakuum zur Trockne ein und trocknet im Vakuum über festem Natriumhydroxid bei 90 °C.
Ausbeute: 0,5 g (90 % der Theorie),
$R_f$-Wert: 0,71 (Reversed Phase Platte RP18; Methanol/5%ige Natriumchloridlösung = 6:4)
Analog werden folgende Verbindungen erhalten:

(1) 5-[(2-Carboxy-ethyl)-aminocarbonyl]-1-methyl-2-[(4-piperidinyl)-aminocarbonyl]-benzimidazol-dihydrobromid
$R_f$-Wert: 0,72 (Reversed Phase Platte RP18; Methanol/5%ige Natriumchloridlösung = 6:4)
(2) 5-[(2-Carboxy-ethyl)-aminocarbonyl]-1-methyl-2-[2-(1-methyl-4-piperidinyl)-ethyl]-benzimidazol-dihydrobromid
(3) 5-[(2-Carboxy-ethyl)-aminocarbonyl]-1-methyl-2-[1-(4-piperidinyl)-2-propyl]-benzimidazol-dihydrobromid
(4) 5-[(2-Carboxy-ethyl)-aminocarbonyl]-1-methyl-2-[2-(4-piperidinyl)-propyl]-benzimidazol-dihydrobromid
(5) 8-(4-Amidino-phenyl)-1-(4-carboxy-butyl)-9-cyclohexyl-3-methyl-xanthin-dihydrobromid
(6) 5-[(2-Carboxy-ethyl)-aminocarbonyl]-1-methyl-2-[(4-piperidinyl)-aminomethyl]-benzimidazol-dihydrobromid
(7) 5-[(2-Carboxy-ethyl)-aminocarbonyl]-1-methyl-2-[N-methyl-N-(4-piperidinylmethyl)-amino]-benzimidazol-dihydrobromid

(8) 5-[(2-Carboxy-ethyl)-aminocarbonyl]-1-methyl-2-[[N-methyl-N-(4-piperidinyl)-amino]-methyl]-benzimidazol-trihydrobromid

(9) 5-[(2-Carboxy-ethyl)-aminocarbonyl]-1-methyl-2-[(4-piperidinyl)-methylthio]-benzimidazol-dihydrobromid

(10) 5-[(2-Carboxy-ethyl)-aminocarbonyl]-1-methyl-2-[4-(4-piperidinyl)-butyl]-benzimidazol-dihydrobromid

(11) 5-[(2-Carboxy-ethyl)-aminocarbonyl]-1-methyl-2-[3-(4-piperidinyl)-propyl]-benzimidazol-dihydrobromid

(12) 5-[(2-Carboxy-ethyl)-aminocarbonyl]-1-methyl-2-(2-piperazino-ethyl)-benzimidazol-trihydrobromid

(13) 5-[(2-Carboxy-ethyl)-aminocarbonyl]-1-methyl-2-[2-(2-methyl-piperazino)-ethyl]-benzimidazol-trihydrobromid

(14) 5-(4-Carboxy-butyl)-1-methyl-2-[2-(4-piperidinyl)-ethyl]-benzimidazol-dihydrobromid

(15) 5-(4-Carboxy-1-oxo-butyl)-1-methyl-2-[2-(4-piperidinyl)-ethyl]-benzimidazol-dihydrobromid

$R_f$-Wert: 0,66 (Reversed Phase Platte RP18; Methanol/5%ige Natriumchloridlösung = 6:4)

(16) 5-[(2-Carboxy-propyl)-aminocarbonyl]-1-methyl-2-[2-(4-piperidinyl)-ethyl]-benzimidazol-dihydrobromid

(17) 5-[(3-Carboxy-propyl)-aminocarbonyl]-1-methyl-2-[2-(4-piperidinyl)-ethyl]-benzimidazol-dihydrobromid

(18) 5-[(2-Carboxy-ethyl)-aminocarbonyl]-1-cyclohexyl-2-[2-(4-piperidinyl)-ethyl]-benzimidazol-dihydrobromid

(19) 5-[(2-Carboxy-ethyl)-aminocarbonyl]-1-methyl-6-methylamino-2-[2-(4-piperidinyl)-ethyl]-benzimidazol-dihydrobromid

(20) 5-[(2-Carboxy-ethyl)-aminocarbonyl]-6-dimethylamino-1-methyl-2-[2-(4-piperidinyl)-ethyl]-benzimidazol-dihydrobromid

(21) 5-[(2-(Carboxy-ethyl)-aminocarbonyl]-6-hydroxy-1-methyl-2-[2-(4-piperidinyl)-ethyl]-benzimidazol-dihydrobromid

(22) 6-Amino-5-[(2-carboxy-ethyl)-aminocarbonyl]-1-methyl-2-[2-(4-piperidinyl)-ethyl]-benzimidazol-dihydrobromid

(23) 5(6)-[(2-Carboxy-ethyl)-aminocarbonyl]-2-[2-(4-piperidinyl)-ethyl]-benzimidazol-dihydrobromid

(24) 6-[(2-Carboxy-ethyl)-aminocarbonyl]-1-methyl-2-[2-(4-piperidinyl)-ethyl]-benzimidazol-dihydrobromid

(25) 5-[(2-Carboxy-ethyl)-aminocarbonyl]-1-(4-phenyl-butyl)-2-[2-(4-piperidinyl)-ethyl]-benzimidazol-dihydrobromid

(26) 6-(3-Carboxy-propylsulfonyl)-1-methyl-2-[2-(4-piperidinyl)-ethyl]-benzimidazol-dihydrobromid

(27) 1-n-Butyl-5-[(2-Carboxy-ethyl)-aminocarbonyl]-2-[2-(4-piperidinyl)-ethyl]-benzimidazol-dihydrobromid

(28) 5-[(2-Carboxy-ethyl)-aminocarbonyl]-1-(3-morpholino-propyl)-2-[2-(4-piperidinyl)-ethyl]-benzimidazol-trihydrobromid

(29) 5-[(2-Carboxy-ethyl)-aminocarbonyl]-1-[3-(4-methyl-piperazino)-propyl]-2-[2-(4-piperidinyl)-ethyl]-benzimidazol-trihydrobromid

(30) 5-[(2-Carboxy-ethyl)-aminocarbonyl]-1-(3-piperazino-propyl)-2-[2-(4-piperidinyl)-ethyl]-benzimidazol-trihydrobromid

(31) 1-(2-Amino-ethyl)-5-(2-carboxy-ethyl)-aminocarbonyl]-2-[2-(4-piperidinyl)-ethyl]-benzimidazol-trihydrobromid

(32) 5-[(2-Carboxy-ethyl)-aminocarbonyl]-2-[2-(4-piperidinyl)-ethyl]-benzoxazol-hydrobromid

(33) 6-[(2-Carboxy-ethyl)-aminocarbonyl]-3-methyl-2-[2-(4-piperidinyl)-ethyl]-imidazo[4,5-b]pyridin-dihydrobromid

(34) 5-[(2-Carboxy-ethyl)-aminocarbonyl]-1-methyl-2-[2-(4-pyridyl)-ethyl]-benzimidazol-dihydrobromid

(35) 5-[(2-Carboxy-ethyl)-aminocarbonyl]-2-(3-imidazolyl-propyl)-1-methyl-benzimidazol-dihydrobromid

(36) 5-[(2-Carboxy-ethyl)-carbonylamino]-1-methyl-2-[2-(4-piperidinyl)-ethyl]-benzimidazol-dihydrobromid

(37) 5-[(2-Carboxy-ethyl)-aminosulfonyl]-1-methyl-2-[2-(4-piperidinyl)-ethyl]-benzimidazol-dihydrobromid

(38) 6-(3-Carboxy-propylthio)-1-methyl-2-[2-(4-piperidinyl)-ethyl]-benzimidazol-dihydrobromid

(39) 8-(4-Amidino-phenyl)-1-(4-carboxy-butyl)-3-methyl-9-(3-phenyl-propyl)-xanthin-dihydrobromid

(40) 8-(4-Amidino-phenyl)-1-(4-carboxy-butyl)-9-isobutyl-3-methyl-xanthin-dihydrobromid

(41) 8-(4-Amidino-phenyl)-1-(4-carboxy-butyl)-3-methyl-9-(2-morpholino-ethyl)-xanthin-trihydrobromid

(42) 8-(4-Amidino-phenyl)-3-benzyl-1-(4-carboxy-butyl)-9-methyl-xanthin-dihydrobromid

(43) 8-(4-Amidino-phenyl)-1-(4-carboxy-butyl)-9-methyl-3-(3-phenyl-propyl)-xanthin-dihydrobromid

(44) 8-(4-Amidino-phenyl)-3-n-butyl-1-(4-carboxy-butyl)-9-methyl-xanthin-dihydrobromid

(45) 6-(3-Carboxy-propyloxy)-1-methyl-2-[2-(4-piperidinyl)-ethyl]-benzimidazol-dihydrobromid

(46) 8-(4-Amidino-phenyl)-1-(4-carboxy-n-pentyl)-3,9-dimethyl-xanthin-dihydrobromid

(47) 8-(4-Amidino-phenyl)-1-(4-carboxy-butyl)-9-isopropyl-xanthin-dihydrobromid

(48) 1-(4-Carboxy-butyl)-3-methyl-8-[2-(4-piperidinyl)ethyl]-xanthin-dihydrochlorid
Man erhitzt mit 6N Salzsäure eine Stunde auf dem Dampfbad. Schmelzpunkt: 211 ° C (Zers.)
$R_f$-Wert: 0,61 (Reversed Phase Platte RP18; Methanol/5%ige Natriumchloridlösung = 6:4)
(49) 1-(4-Carboxy-butyl)-3,9-dimethyl-8-[2-(4-piperidinyl)-ethyl]-xanthin-dihydrobromid
$R_f$-Wert: 0,70 (Reversed Phase Platte RP18; Methanol/5%ige Natriumchloridlösung = 6:4)
(50) 1-(4-Carboxy-butyl)-9-isopropyl-8-[2-(4-piperidinyl)-ethyl]-xanthin-dihydrobromid
Als Ausgangsmaterial dient 8-[2-(1-Benzyloxycarbonyl-4-piperidinyl)-ethyl]-1-(4-ethoxycarbonyl-butyl)-2-[-(4-ethoxycarbonylbutyl)-oxy]-9-isopropyl-1H-purin-6-on
$R_f$-Wert: 0,59 (Reversed Phase Platte RP18; Methanol/5%ige Natriumchloridlösung = 6:4)
(51) 1-(3-Carboxy-propyl)-3,9-dimethyl-8-[2-(4-piperidinyl)-ethyl]-xanthin-dihydrobromid
(52) 1-(5-Carboxy-pentyl)-3,9-dimethyl-8-[2-(4-piperidinyl)-ethyl]-xanthin-dihydrobromid
(53) 1-(4-Carboxy-butyl)-3-methyl-9-(2-morpholino-ethyl)-8-[2-(4-piperidinyl)-ethyl]-xanthin-trihydrobromid
(54) 8-(4-Carboxy-butyl)-2-[2-(4-piperidinyl)-ethyl]-7,9-dioxo-4,5-dihydro-8H-imidazo[1,2,3-cd]purin-dihydrobromid
(55) 1-(4-Carboxy-butyl)-3-methyl-8-[2-(4-piperidinyl)-ethyl]-xanthin-dihydrobromid
(56) 3-n-Butyl-1-(4-carboxy-butyl)-9-methyl-8-[2-(4-piperidinyl)-ethyl]-xanthin-dihydrobromid
(57) 1-(4-Carboxy-butyl)-3-carboxymethyl-9-methyl-8-[2-(4-piperidinyl)-ethyl]-xanthin-dihydrobromid
(58) 3-Aminocarbonylmethyl-1-(4-carboxy-butyl)-9-methyl-8-[2-(4-piperidinyl)-ethyl]-xanthin-dihydrobromid
(59) 1-(4-Carboxy-butyl)-3-isopropyl-7-methyl-8-[2-(4-piperidinyl)-ethyl]-xanthin-dihydrobromid
(60) 1-(4-Carboxy-butyl)-9-methyl-8-[2-(4-piperidinyl)-ethyl]-hypoxanthin-dihydrobromid
(61) 2-(4-Amidino-phenyl)-9-(4-carboxy-butyl)-8,10-dioxo-5,6-dihydro-4H,9H-pyrimido[1,2,3-cd]purin-dihydrobromid
Schmelzpunkt: sintert ab 40 ° C
$R_f$-Wert: 0,68 (Reversed Phase Platte RP18; Methanol/5%ige Natriumchloridlösung = 6:4)
(62) 2-(4-Amidino-phenyl)-8-(4-carboxy-butyl)-7,9-dioxo-4,5-dihydro-8H-imidazo[1,2,3-cd]purin-dihydrobromid
(63) 9-(4-Carboxy-butyl)-2-[2-(4-piperidinyl)-ethyl]-8,10-dioxo-5,6-dihydro-4H,9H-pyrimido[1,2,3-cd]purin-dihydrobromid
$R_f$-Wert: 0,72 (Reversed Phase Platte RP18; Methanol/5%ige Natriumchloridlösung = 6:4)
(64) 8-(trans-4-Aminomethyl-cyclohexyl)-1-(4-carboxy-butyl)-3-methyl-xanthin-dihydrochlorid
Man geht aus von 8-[trans-4-(Benzyloxycarbonylamino-methyl)-cyclohexyl]-1-(4-ethoxycarbonyl-butyl)-3-methyl-xanthin und verfährt analog (48).
Schmelzpunkt: 248 ° C (Zers.)
$R_f$-Wert: 0,59 (Reversed Phase Platte RP18; Methanol/5%-ige Natriumchloridlösung = 6:4)
(65) 1-(2-Carboxy-ethyl)-3,9-dimethyl-8-[4-(4-piperidinyl)-phenyl]-xanthin-dihydrochlorid
Man verfährt analog (64).
Schmelzpunkt: 122 ° C (Zers.)
$R_f$-Wert: 0,61 (Reversed Phase Platte RP18; Methanol/5%ige Natriumchloridlösung = 6:4)
(66) 8-(4-Amidino-phenyl)-1-(4-carboxy-butyl)-9-methyl-xanthin-dihydrobromid
$R_f$-Wert: 0,48 (Reversed Phase Platte RP18; Methanol/5%ige Natriumchloridlösung = 6:4)
(67) 5-(2-Carboxy-ethenyl)-1-methyl-2-[4-(4-piperidinyl)-phenyl]-benzimidazol-dihydrobromid
Schmelzpunkt: über 250 ° C
$R_f$-Wert: 0,67 (Reversed Phase Platte RP18; Methanol/5%ige Natriumchloridlösung = 6:4)
(68) 5-(2-Carboxy-ethyl)-1-methyl-2-[4-(4-piperidinyl)-phenyl]-benzimidazol-dihydrobromid
Schmelzpunkt: 256 ° C (Zers.)
$R_f$-Wert: 0,67 (Reversed Phase Platte RP18; Methanol/5%ige Natriumchloridlösung = 6:4)
(69) 5-(2-Carboxy-ethyl)-1-methyl-2-(4-piperazino-phenyl)-benzimidazol-trihydrobromid
(70) 8-(4-Amidino-phenyl)-1-(4-carboxy-butyl)-2-chlor-9-methyl-hypoxanthin-dihydrochlorid
Man verwendet konzentrierte Salzsäure und arbeitet bei Raumtemperatur.
$R_f$-Wert: 0,54 (Reversed Phase Platte RP18; Methanol/5%ige Natriumchloridlösung = 6:4)
(71) 8-(4-Amidino-phenyl)-1-(4-carboxy-butyl)-9-methyl-hypoxanthin-dihydrochlorid
Man verfährt analog (70)
$R_f$-Wert: 0,71 (Reversed Phase Platte RP18; Methanol/5%ige Natriumchloridlösung = 6:4)

Beispiel 2

5- [(2-Methoxycarbonyl-ethyl)-aminocarbonyl]-1-methyl-2-[2-(4-piperidinyl)-ethyl]-benzimidazol

1 g 2-[2-(1-Benzyloxycarbonyl-4-piperidinyl)-ethyl]-5-[(2-methoxycarbonyl-ethyl)-aminocarbonyl]-1-methyl-benzimidazol
werden in 20 ml Methanol in Gegenwart von 0,1 g 10%iger Palladiumkohle mit Wasserstoff von 4 bar 6 Stunden bei Raumtemperatur behandelt. Nach Abfiltrieren des Katalysators wird im Vakuum eingedampft.
Ausbeute: 0,7 g (97 % der Theorie),
$R_f$-Wert: 0,68 (Reversed Phase Platte RP18, Methanol/5%ige Natriumchloridlösung = 6:4)
Analog werden folgende Verbindungen erhalten:

(1) 5-[(2-Methoxycarbonyl-ethyl)-aminocarbonyl]-1-methyl-2-[(4-piperidinyl)-aminocarbonyl]-benzimidazol
Aus 2-[(1-Benzyl-4-piperidinyl)-aminocarbonyl]-5-[(2-methoxycarbonyl-ethyl)-aminocarbonyl]-1-methyl-benzimidazol unter Verwendung von 20%iger Palladiumkohle und unter Zusatz von methanolischer Salzsäure.
$R_f$-Wert: 0,56 (Reversed Phase Platte RP18, Methanol/5%ige Natriumchloridlösung = 6:4)
(2) 5-[(2-Methoxycarbonyl-ethyl)-aminocarbonyl]-1-methyl-2-[1-(4-piperidinyl)-2-propyl]-benzimidazol
(3) 5-[(2-Methoxycarbonyl-ethyl)-aminocarbonyl]-1-methyl-2-[2-(4-piperidinyl)-propyl]-benzimidazol
(4) 5-[(2-Methoxycarbonyl-ethyl)-aminocarbonyl]-1-methyl-2-[(4-piperidinyl)-oxymethyl]-benzimidazol
(5) 5-[(2-Methoxycarbonyl-ethyl)-aminocarbonyl]-1-methyl-2-[(4-piperidinyl)-aminomethyl]-benzimidazol
(6) 5-[(2-Methoxycarbonyl-ethyl)-aminocarbonyl]-1-methyl-2-[N-methyl-N-(4-piperidinylmethyl)-amino]-benzimidazol
(7) 5-[(2-Methoxycarbonyl-ethyl)-aminocarbonyl]-1-methyl-2-[[N-methyl-N-(4-piperidinyl)-amino]-methyl]-benzimidazol
(8) 5-[(2-Methoxycarbonyl-ethyl)-aminocarbonyl]-1-methyl-2-[(4-piperidinyl)-methylthio]-benzimidazol
(9) 5-[(2-Methoxycarbonyl-ethyl)-aminocarbonyl]-1-methyl-2-[4-(4-piperidinyl)-butyl]-benzimidazol
(10) 5-[(2-Methoxycarbonyl-ethyl)-aminocarbonyl]-1-methyl-2-[3-(4-piperidinyl)-propyl]-benzimidazol
(11) 5-[(2-Methoxycarbonyl-ethyl)-aminocarbonyl]-1-methyl-2-(2-piperazino-ethyl)-benzimidazol
(12) 5-[(2-Methoxycarbonyl-ethyl)-aminocarbonyl]-1-methyl-2-[2-(2-methyl-piperazino)-ethyl]-benzimidazol
(13) 5-(4-Methoxycarbonyl-butyl)-1-methyl-2-[2-(4-piperidinyl)-ethyl]-benzimidazol
(14) 5-(4-Isopropyloxycarbonyl-1-oxo-butyl)-1-methyl-2-[2-(4-piperidinyl)-ethyl]-benzimidazol
$R_f$-Wert: 0,31 (Reversed Phase Platte RP18; Methanol/5%ige Natriumchloridlösung = 6:4)
(15) 5-[(2-Methoxycarbonyl-propyl)-aminocarbonyl]-1-methyl-2-[2-(4-piperidinyl)-ethyl]-benzimidazol
(16) 5-[(3-Methoxycarbonyl-propyl)-aminocarbonyl]-1-methyl-2-[2-(4-piperidinyl)-ethyl]-benzimidazol
(17) 6-Methoxy-5-[(2-methoxycarbonyl-ethyl)-aminocarbonyl]-1-methyl-2-[2-(4-piperidinyl)-ethyl]-benzimidazol
(18) 5-[(2-Methoxycarbonyl-ethyl)-aminocarbonyl]-1-methyl-6-methylamino-2-[2-(4-piperidinyl)-ethyl]-benzimidazol
(19) 6-Dimethylamino-5-[(2-methoxycarbonyl-ethyl)-aminocarbonyl]-1-methyl-2-[2-(4-piperidinyl)-ethyl]-benzimidazol
(20) 6-Hydroxy-5-[(2-methoxycarbonyl-ethyl)-aminocarbonyl]-1-methyl-2-[2-(4-piperidinyl)-ethyl]-benzimidazol
(21) 6-Amino-5-[(2-methoxycarbonyl-ethyl)-aminocarbonyl]-1-methyl-2-[2-(4-piperidinyl)-ethyl]-benzimidazol
(22) 5(6)-[(2-Methoxycarbonyl-ethyl)-aminocarbonyl]-2-[2-(4-piperidinyl)-ethyl]-benzimidazol
(23) 6-[(2-Methoxycarbonyl-ethyl)-aminocarbonyl]-1-methyl-2-[2-(4-piperidinyl)-ethyl]-benzimidazol
(24) 5-[(2-Methoxycarbonyl-ethyl)-aminocarbonyl]-1-(4-phenylbutyl)-2-[2-(4-piperidinyl)-ethyl]-benzimidazol
(25) 1-[2-(3,4-Dimethoxy-phenyl)-ethyl]-5-[(2-methoxycarbonyl-ethyl)-aminocarbonyl]-2-[2-(4-piperidinyl)-ethyl]-benzimidazol
(26) 1-n-Butyl-5-[(2-methoxycarbonyl-ethyl)-aminocarbonyl]-2-[2-(4-piperidinyl)-ethyl]-benzimidazol
(27) 5-[(2-Methoxycarbonyl-ethyl)-aminocarbonyl]-1-(3-morpholino-propyl)-2-[2-(4-piperidinyl)-ethyl]-benzimidazol
(28) 5-[(2-Methoxycarbonyl-ethyl)-aminocarbonyl]-1-[3-(4-methyl-piperazino)-propyl]-2-[2-(4-piperidinyl)-ethyl]-benzimidazol
(29) 5-[(2-Methoxycarbonyl-ethyl)-aminocarbonyl]-1-(3-piperazino-propyl)-2-[2-(4-piperidinyl)-ethyl]-benzimidazol

(30) 1-(2-Amino-ethyl)-5-[(2-methoxycarbonyl-ethyl)-aminocarbonyl]-2-[2-(4-piperidinyl)-ethyl]-benzimidazol
(31) 5-[2-Methoxycarbonyl-ethyl)-aminocarbonyl]-2-[2-(4-piperidinyl)-ethyl]-benzoxazol
(32) 6-[(2-Methoxycarbonyl-ethyl)-aminocarbonyl]-3-methyl-2-[2-(4-piperidinyl)-ethyl]-imidazo[4,5-b]-pyridin
(33) 5-[(2-Methoxycarbonyl-ethyl)-carbonylamino]-1-methyl-2-[2-(4-piperidinyl)-ethyl]-benzimidazol
(34) 5-[(2-Methoxycarbonyl-ethyl)-aminosulfonyl]-1-methyl-2-[2-(4-piperidinyl)-ethyl]-benzimidazol
(35) 3,9-Dimethyl-1-(4-ethoxycarbonyl-butyl)-8-[2-(4-piperidinyl)-ethyl]-xanthin
$R_f$-Wert: 0,41 (Reversed Phase Platte RP18; Methanol/5%ige Natriumchloridlösung = 6:4)
(36) 3,7-Dimethyl-1-(4-ethoxycarbonyl-butyl)-8-[2-(4-piperidinyl)-ethyl]-xanthin
Schmelzpunkt: 206-208 ° C (sintert ab 205 ° C)
$R_f$-Wert: 0,34 (Reversed Phase Platte RP18; Methanol/5%ige Natriumchloridlösung = 6:4)
(37) 3,9-Dimethyl-1-(3-methoxycarbonyl-propyl)-8-[2-(4-piperidinyl)-ethyl]-xanthin
(38) 3,9-Dimethyl-1-(5-methoxycarbonyl-pentyl)-8-[2-(4-piperidinyl)-ethyl]-xanthin
(39) 1-(4-Methoxycarbonyl-butyl)-3-methyl-9-(2-morpholino-ethyl)-8-[2-(4-piperidinyl)-ethyl]-xanthin
(40) 1-(4-Methoxycarbonyl-butyl)-9-(2-methoxy-ethyl)-3-methyl-8-[2-(4-piperidinyl)-ethyl]-xanthin
(41) 1-(4-Methoxycarbonyl-butyl)-3-methyl-8-[2-(4-piperidinyl)-ethyl]-xanthin
(42) 3-n-Butyl-1-(4-methoxycarbonyl-butyl)-9-methyl-8-[2-(4-piperidinyl)-ethyl]-xanthin
(43) 1-(4-Methoxycarbonyl-butyl)-3-methoxycarbonylmethyl-9-methyl-8-[2-(4-piperidinyl)-ethyl]-xanthin
(44) 3-Aminocarbonylmethyl-1-(4-methoxycarbonyl-butyl)-9-methyl-8-[2-(4-piperidinyl)-ethyl]-xanthin
(45) 3-Isopropyl-1-(4-methoxycarbonyl-butyl)-7-methyl-8-[2-(4-piperidinyl)-ethyl]-xanthin
(46) 1-(4-Methoxycarbonyl-butyl)-9-methyl-8-[2-(4-piperidinyl)-ethyl]-hypoxanthin
(47) 9-(4-Ethoxycarbonyl-butyl)-2-[2-(4-piperidinyl)-ethyl]-8,10-dioxo-5,6-dihydro-4H,9H-pyrimido[1,2,3-cd]purin $R_f$-Wert: 0,03 (Kieselgel; Essigester/Ethanol = 7:3)
(48) 8-(4-Methoxycarbonyl-butyl)-2-[2-(4-piperidinyl)-ethyl]-7,9-dioxo-8H-imidazo[1,2,3-cd]purin
(49) 6-(3-Methoxycarbonyl-propyloxy)-1-methyl-2-[2-(4-piperidinyl)-ethyl]-benzimidazol
(50) 6-(3-Methoxycarbonyl-propylthio)-1-methyl-2-[2-(4-piperidinyl)-ethyl]-benzimidazol
(51) 6-(3-Methoxycarbonyl-propylsulfonyl)-1-methyl-2-[2-(4-piperidinyl)-ethyl]-benzimidazol
(52) 1-Cyclopropyl-5-[(2-methoxycarbonyl-ethyl)-aminocarbonyl]-2-[2-(4-piperidinyl)-ethyl]-benzimidazol
(53) 1-Cyclohexyl-5-[(2-methoxycarbonyl-ethyl)-aminocarbonyl]-2-[2-(4-piperidinyl)-ethyl]-benzimidazol
(54) 9-Cyclopropyl-1-(4-methoxycarbonyl-butyl)-3-methyl-8-[2-(4-piperidinyl)-ethyl]-xanthin
(55) 1-(4-Ethoxycarbonyl-butyl)-3-methyl-8-[2-(4-piperidinyl)-ethyl]-xanthin-dihydrochlorid
Man arbeitet in Ethanol.
Schmelzpunkt: 185 ° C (Zers., beginnt bei 155 ° C)
$R_f$-Wert: 0,38 (Kieselgel; Methylenchlorid/Methanol/Eisessig = 8:2:0,1)

## Beispiel 3

## 2-[2-(1-Benzyloxycarbonyl-4-piperidinyl)-ethyl]-5-[(2-methoxycarbonyl-ethyl)-aminocarbonyl]-1-methyl-benzimidazol

Eine Mischung aus 2,5 g 3-Amino-4-methylamino-benzoesäure-[N-(2-methoxycarbonyl-ethyl)-amid] [$R_f$-Wert: 0,28 (Kieselgel; Essigester/Ethanol = 50:2); hergestellt aus 4-Chlor-3-nitro-benzoylchlorid und β-Alanin durch Umsetzung in 1N Natronlauge zu 4-Chlor-3-nitro-benzoesäure-[N-(2-carboxy-ethyl)-amid] (Schmelzpunkt: 165-167 ° C), Umsetzung dieses Produktes mit 40%iger wäßriger Methylaminlösung bei 50 ° C in einem Druckgefäß zu 4-Methylamino-3-nitro-benzoesäure-[N-(2-carboxy-ethyl)-amid] (Schmelzpunkt: 187-189 ° C), Veresterung mit Methanol/etherischer Salzsäure zu 4-Methylamino-3-nitro-benzoesäure-[N-(2-methoxycarbonyl-ethyl)-amid] (Schmelzpunkt: 125-127 ° C) und Reduktion mit Wasserstoff in Gegenwart von 10%iger Palladiumkohle in Methanol bei 3 bar und Raumtemperatur], 50 ml Methylenchlorid, 1,74 ml N-Ethyl-diisopropylamin und 0,1 g 4-Dimethylamino-pyridin wird mit 3,1 g 3-(1-Benzyloxycarbonyl-4-piperidinyl)-propionylchlorid versetzt und 16 Stunden bei Raumtemperatur gerührt. Man dampft im Vakuum ein, nimmt mit Essigester/verdünnter Salzsäure auf und extrahiert die wäßrige Phase noch zweimal mit Essigester. Die Essigesterphasen werden eingedampft, der Rückstand in 30 ml Eisessig aufgenommen und 1,5 Stunden auf dem Dampfbad erwärmt. Man dampft im Vakuum ein, versetzt mit Wasser und neutralisiert durch Zusatz von Natriumbicarbonat. Man extrahiert mit Essigester, dampft die organischen Phasen ein und reinigt den Rückstand durch Chromatographie an Kieselgel (Elutionsmittel: Essigester/Ethanol = 100:1 bis 6:4).
Ausbeute: 1,2 g (24 % der Theorie),

$R_f$-Wert: 0,63 (Kieselgel; Essigester/Ethanol = 8:2)
Analog werden folgende Verbindungen erhalten:
(1) 5-[(2-Methoxycarbonyl-ethyl)-aminocarbonyl]-2-[2-(1-methoxycarbonyl-4-piperidinyl)-ethyl]-1-methyl-benzimidazol
(2) 5-[(2-Methoxycarbonyl-ethyl)-aminocarbonyl]-1-methyl-2-[2-(4-pyridyl)-ethyl]-benzimidazol
(3) 2-[3-(1-Imidazolyl)-propyl]-5-[(2-methoxycarbonyl-ethyl)-aminocarbonyl]-1-methyl-benzimidazol
(4) 2-[(1-Benzyloxycarbonyl-4-piperidinyl)-ethyl]-5-(4-isopropyloxycarbonyl-1-oxo-butyl)-1-methyl-benzimidazol $R_f$-Wert: 0,62 (Kieselgel; Essigester/Ethanol = 9:1)
(5) 5-[(2-Methoxycarbonyl-ethyl)-aminocarbonyl]-1-methyl-2-[2-(1-methyl-4-piperidinyl)-ethyl]-benzimidazol

Beispiel 4

2-[(1-Benzyl-4-piperidinyl)-aminocarbonyl]-5-[(2-methoxycarbonyl-ethyl)-aminocarbonyl]-1-methyl-benzimidazol

Eine Mischung aus 3,78 g 5-[(2-Methoxycarbonyl-ethyl)-aminocarbonyl]-1-methyl-2-trichlormethyl-benzimidazol, 1,9 g 4-Amino-1-benzyl-piperidin, 2,5 g Natriumbicarbonat und 40 ml Wasser wird 10 Stunden auf dem Dampfbad erwärmt. Man filtriert das vorhandene Festprodukt ab, wäscht es mit Wasser und kristallisiert aus Essigester um.
Ausbeute: 1,0 g (22 % der Theorie),
Schmelzpunkt: 168-170 °C
$R_f$-Wert: 0,32 (Kieselgel; Essigester/Ethanol = 7:3)

Beispiel 5

8-(4-Amidino-phenyl)-3,9-dimethyl-1-(4-isopropyloxycarbonyl-butyl)-xanthin-dihydrochlorid

0,15 g 8-(4-Amidino-phenyl)-1-(4-carboxy-butyl)-3,9-dimethyl-xanthin werden in 30 ml Isopropanol suspendiert. Man leitet 60 Minuten lang Salzsäuregas ein und rührt noch zwei Stunden nach. Man dampft zur Trockne ein und verreibt den Rückstand mit Ether, wodurch man ein amorphes Festprodukt erhält.
Ausbeute: 0,14 g (72 % der Theorie),
$R_f$-Wert: 0,38 (Kieselgel; n-Butanol/Eisessig/Wasser = 4:1:2)
Analog werden folgende Verbindungen erhalten:
(1) 5-[(2-Isobutyloxycarbonyl-ethyl)-aminocarbonyl]-1-methyl-2-[2-(4-piperidinyl)-ethyl]-benzimidazol
(2) 5-[(2-Cyclohexyloxycarbonyl-ethyl)-aminocarbonyl]-1-methyl-2-[2-(4-piperidinyl)-ethyl]-benzimidazol
(3) 8-(4-Amidino-phenyl)-1-(4-cyclohexyloxycarbonyl-butyl)-3,9-dimethyl-xanthin
(4) 8-(4-Amidino-phenyl)-3,9-dimethyl-1-(4-ethoxycarbonyl-butyl)-xanthin
(5) 8-(trans-4-Aminomethyl-cyclohexyl)-1-(4-methoxycarbonyl-butyl)-3-methyl-xanthin-dihydrochlorid
Man arbeitet in Methanol unter Zusatz etherischer Salzsäure.
Schmelzpunkt: 264-266 °C (Zers.)
$R_f$-Wert: 0,49 (Reversed Phase Platte RP18; Methanol/5%ige Natriumchloridlösung = 6:4)
(6) 3,9-Dimethyl-1-(2-methoxy-carbonyl-ethyl)-8-[4-(4-piperidinyl)-phenyl]-xanthin-dihydrochlorid.
Schmelzpunkt: 120 °C (Zers.)
$R_f$-Wert: 0,18 (Reversed Phase Platte RP18; Methanol/5%ige Natriumchloridlösung = 6:4)

Beispiel 6

8-(4-Amidino-phenyl)-1-(4-carboxy-butyl)-3,9-dimethyl-xanthin

0,4 g 8-(4-Amidino-phenyl)-3,9-dimethyl-(4-methoxycarbonyl-butyl)-xanthin, 5 ml Wasser, 5 ml 1N Lithiumhydroxid und 5 ml Tetrahydrofuran werden 60 Minuten bei Raumtemperatur gerührt. Man dampft das Tetrahydrofuran im Vakuum ab und fügt 0,27 g Ammonchlorid zu, wonach das Reaktionsgemisch im Vakuum auf die Hälfte seines Volumens eingedampft wird. Das ausgefallene Festprodukt wird mit wenig Wasser, dann Aceton und Ether gewaschen.
Ausbeute: 0,35 g (99 % der Theorie),
Schmelzpunkt: 263-265 °C (Zers.)
$R_f$-Wert: 0,49 (Kieselgel; Butanol/Eisessig/Wasser = 4:1:1,5)

Analog werden folgende Verbindungen erhalten:

(1) 5-[(2-Carboxy-ethyl)-aminocarbonyl]-1-methyl-2-[(4-piperidinyl)-oxymethyl]-benzimidazol

(2) 5-[(2-Carboxy-ethyl)-aminocarbonyl]-6-methoxy-1-methyl-2-[(4-piperidinyl)-ethyl]-benzimidazol

(3) 5-[(2-Carboxy-ethyl)-aminocarbonyl]-1-[2-(3,4-dimethoxy-phenyl)-ethyl]-2-[2-(4-piperidinyl)-ethyl]-benzimidazol

(4) 8-(4-Amidino-phenyl)-1-(4-carboxy-butyl)-9-[2-(3,4-dimethoxy-phenyl)-ethyl]-3-methyl-xanthin

(5) 8-(4-Amidino-phenyl)-1-(4-carboxy-butyl)-3-(3-methoxypropyl)-9-methyl-xanthin

(6) 1-(4-Carboxy-butyl)-9-(2-methoxy-ethyl)-3-methyl-8-[2-(4-piperidinyl)-ethyl]-xanthin

(7) 6-(3-Carboxy-propylsulfinyl)-1-methyl-[2-(4-piperidinyl)-ethyl]-benzimidazol

(8) 5-[(2-Carboxy-ethyl)-aminocarbonyl]-1-cyclopropyl-2-[2-(4-piperidinyl)-ethyl]-benzimidazol

(9) 8-(4-Amidino-phenyl)-1-(4-carboxy-butyl)-9-cyclopropyl-3-methyl-xanthin

(10) 1-(4-Carboxy-butyl)-9-cyclopropyl-3-methyl-8-[2-(4-piperidinyl)-ethyl]-xanthin

(11) 8-(4-Amidino-phenyl)-1-(4-carboxy-butyl)-3,7-dimethyl-xanthin-dihydrobromid
Schmelzpunkt: 290-292 °C (sintert ab 288 °C)
$R_f$-Wert: 0,68 (Reversed Phase Platte RP18; Methanol/5%ige Natriumchloridlösung = 6:4, nach zweimaliger Entwicklung)

(12) 1-(4-Carboxy-butyl)-3,7-dimethyl-8-[2-(4-piperidinyl)-ethyl]-xanthin
Schmelzpunkt: 278-280 °C (sintert ab 270 °C)
$R_f$-Wert: 0,57 (Reversed Phase Platte RP18; Methanol/5%ige Natriumchloridlösung = 6:4)

Beispiel 7

8-(4-Amidino-phenyl)-3,9-dimethyl-1-(4-methoxycarbonyl-butyl)-xanthin-hydrochlorid-hydrogenacetat

3,3 g 8-(4-Cyano-phenyl)-3,9-dimethyl-1-(4-ethoxycarbonyl-butyl)-xanthin werden in 150 ml Methanol suspendiert und auf -30 °C gekühlt. Man leitet 30 Minuten lang Salzsäuregas ein, wobei man die Temperatur zwischen -5 und -10 °C hält, und läßt 16 Stunden bei Raumtemperatur stehen. Man dampft im Vakuum zur Trockne ein und dampft nochmals mit Methanol nach. Der Rückstand wird in 150 ml Methanol gelöst und portionsweise mit Ammoncarbonat versetzt. Man rührt 16 Stunden bei Raumtemperatur nach, säuert mit etherischer Salzsäure schwach an und dampft im Vakuum zur Trockne ein. Man verreibt mit Methylenchlorid und reinigt das erhaltene Festprodukt durch Chromatographie an Kieselgel (Elutionsmittel: Methanol, dann Methanol/2N Essigsäure = 10:1).
Ausbeute: 1,0 g (24 % der Theorie),
Schmelzpunkt: 224-226 °C (Zers.)
$R_f$-Wert: 0,37 (Kieselgel; n-Butanol/Eisessig/Wasser = 4:1:2)
Analog werden folgende Verbindungen erhalten:

(1) 8-(4-Amidino-phenyl)-9-[2-(3,4-dimethoxy-phenyl)-ethyl]-1-(4-methoxycarbonyl-butyl)-3-methyl-xanthin

(2) 8-(4-Amidino-phenyl)-1-(4-methoxycarbonyl-butyl)-3-methyl-9-(3-phenyl-propyl)-xanthin

(3) 8-(4-Amidino-phenyl)-9-isobutyl-1-(4-methoxycarbonyl-butyl)-3-methyl-xanthin

(4) 8-(4-Amidino-phenyl)-1-(4-methoxycarbonyl-butyl)-3-methyl-9-(2-morpholino-ethyl)-xanthin

(5) 8-(4-Amidino-phenyl)-3-benzyl-1-(4-methoxycarbonyl-butyl)-9-methyl-xanthin

(6) 8-(4-Amidino-phenyl)-1-(4-methoxycarbonyl-butyl)-9-methyl-3-(3-phenyl-propyl)-xanthin

(7) 8-(4-Amidino-phenyl)-3-n-butyl-1-(4-methoxycarbonyl-butyl)-9-methyl-xanthin

(8) 8-(4-Amidino-phenyl)-1-(4-methoxycarbonyl-butyl)-3-(3-methoxy-propyl)-9-methyl-xanthin

(9) 8-(4-Amidino-phenyl)-3,9-dimethyl-1-(4-methoxycarbonyl-pentyl)-xanthin

(10) 8-(4-Amidino-phenyl)-9-isopropyl-1-(4-methoxycarbonyl-butyl)-xanthin

(11) 8-(4-Amidino-phenyl)-3,7-dimethyl-1-(4-methoxycarbonyl-butyl)-xanthin
Schmelzpunkt: 248-250 °C (Zers.)
$R_f$-Wert: 0,43 (Kieselgel; Methylenchlorid/Methanol/konz. Ammoniak = 3:1:0,1, nach zweimaliger Entwicklung)

(12) 2-(4-Amidino-phenyl)-9-(4-methoxycarbonyl-butyl)-8,10-dioxo-5,6-dihydro-4H,9H-pyrimido[1,2,3-cd]-purin
Schmelzpunkt: Zers. ab 105 °C
$R_f$-Wert: 0,38 (Reversed Phase Platte RP18; Methanol/5%ige Natriumchloridlösung = 6:4)

(13) 2-(4-Amidino-phenyl)-8-(4-methoxycarbonyl-butyl)-7,9-dioxo-4,5-dihydro-8H-imidazo[1,2,3-cd]purin

(14) 8-(4-Amidino-phenyl)-9-cyclopropyl-1-(4-methoxycarbonyl-butyl)-3-methyl-xanthin

(15) 8-(4-Amidino-phenyl)-9-cyclohexy1-1-(4-methoxycarbonyl-butyl)-3-methyl-xanthin

(16) 8-(4-Amidino-phenyl)-1-(4-methoxycarbonyl-butyl)-9-methyl-xanthin
Schmelzpunkt: 225 ° C (Zers., sintert ab 202 ° C)
$R_f$-Wert: 0,35 (Reversed Phase Platte RP18; Methanol/5%ige Natriumchloridlösung = 6:4)
(17) 8-(4-Amidino-phenyl)-2-chlor-1-(4-ethoxycarbonyl-butyl)-9-methyl-hypoxanthin
Man verwendet ethanolische Salzsäure.
$R_f$-Wert: 0,30 (Reversed Phase Platte RP18; Methanol/5%ige Natriumchloridlösung = 6:4)

Beispiel 8

3,9-Dimethyl-8-[4-(N-methoxycarbonyl-amidino)-phenyl]-1-(4-methoxycarbonyl-butyl)-xanthin

Herstellung aus 8-(4-Amidino-phenyl)-3,9-dimethyl-1-(4-methoxycarbonyl-butyl)-xanthin und Chlorameisensäure-methylester in Methylenchlorid unter Zugabe von Natronlauge und kräftigem Rühren.

Beispiel 9

8-[2-(1-Acetoxymethoxycarbonyl-4-piperidinyl)-ethyl]-3,9-dimethyl-1-(4-methoxycarbonyl-butyl)-xanthin

Herstellung aus 3,9-Dimethyl-1-(4-methoxycarbonyl-butyl)-8-[2-(4-piperidinyl)-ethyl]-xanthin und Kohlensäure-acetoxymethyl-(4-nitro-phenyl)-ester in Methylenchlorid in Gegenwart von N-Ethyl-diisopropylamin bei Raumtemperatur.

Analog wird folgende Verbindung erhalten:
2-[2-(1-Acetoxymethyloxycarbonyl-4-piperidinyl)-ethyl]-5-[(2-methoxycarbonyl-ethyl)-aminocarbonyl]-1-methyl-benzimidazol

Beispiel 10

6-(3-Methoxycarbonyl-propylsulfinyl)-1-methyl-2-[2-(4-piperidinyl)-ethyl]-benzimidazol

Herstellung aus 6-(3-Methoxycarbonyl-propylthio)-1-methyl-2-[2-(4-piperidinyl)-ethyl]-benzimidazol mit 30%igem Wasserstoffperoxid in Eisessig unter Eiskühlung.

Beispiel 11

8-(4-Cyano-phenyl)-3,9-dimethyl-1-(4-ethoxycarbonyl-butyl)-xanthin

4,5 g 8-(4-Cyano-phenyl)-3,9-dimethyl-xanthin werden bei 80 ° C in Dimethylsulfoxid gelöst, mit 2,3 g Kaliumcarbonat versetzt und 2 Stunden bei 80 ° C gerührt. Man läßt abkühlen, fügt tropfenweise 2,6 ml 5-Brom-valeriansäure-ethylester zu und erwärmt unter Rühren 4 Stunden auf 80 ° C. Man dampft das Lösungsmittel im Vakuum ab und nimmt den Rückstand mit einer Mischung aus 250 ml Essigester und 150 ml Wasser auf. Die wäßrige Phase wird abgetrennt, noch zweimal mit je 100 ml Esssigester extrahiert und die vereinigten organischen Phasen eingedampft. Der Rückstand wird säulenchromatographisch an Kieselgel gereinigt (Elutionsmittel: Essigester).
Ausbeute: 3,4 g (52 % der Theorie),
Schmelzpunkt: 148-150 ° C
$R_f$-Wert: 0,69 (Kieselgel; Methylenchlorid/Methanol/Eisessig = 19:1:0,1)
Analog werden folgende Verbindungen erhalten:
(1) 8-[2-(1-Benzyloxycarbonyl-4-piperidinyl)-ethyl]-3,9-dimethyl-1-(4-ethoxycarbonyl-butyl)-xanthin
Man verwendet Kalium-tert.butylat, Reaktionsdauer: 6 Stunden $R_f$-Wert: 0,24 (Kieselgel; Methylenchlorid/Methanol = 19:1)
(2) 2-[2-(1-Benzyloxycarbonyl-4-piperidinyl)-ethyl]-9-(4-ethoxycarbonyl-butyl)-8,10-dioxo-5,6-dihydro-4H,9H-pyrimido[1,2,3-cd]purin
Man verfährt analog (1).
$R_f$-Wert: 0,84 (Kieselgel; Essigester/Ethanol = 7:3)
(3) 8-[2-(1-Benzyloxycarbonyl-4-piperidinyl)-ethyl]-1-(4-ethoxycarbonyl-butyl)-2-[(4-ethoxycarbonyl-butyl)-oxy]-9-isopropyl-1H-purin-6-on.
Man verwendet Dimethylformamid als Lösungsmittel und setzt zunächst äquimolare Mengen Kalium-tert.butylat, 5-Brom-valeriansäure-ethylester und 8-[2-(1-Benzyloxycarbonyl-4-piperidinyl)-ethyl]-9-isopropyl-xanthin ein, erhitzt 16 Stunden auf 80 °C, fügt je ein Äquivalent 5-Brom-valeriansäure-ethylester und Kaliumkarbonat zu und rührt weitere 5 Stunden bei 80 °C.
$R_f$-Wert: 0,24 (Kieselgel; Methylenchlorid/Methanol = 19:1)
(4) 8-(4-Cyano-phenyl)-3,7-dimethyl-1-(4-ethoxycarbonyl-butyl)-xanthin
Man verwendet Dimethylformamid.
Schmelzpunkt: 98-99 °C
$R_f$-Wert: 0,83 (Kieselgel; Methylenchlorid/Methanol/Eisessig = 19:1:0,1)
(5) 8-[2-(1-Benzyloxycarbonyl-4-piperidinyl)-ethyl]-3,7-dimethyl-1-(4-ethoxycarbonyl-butyl)-xanthin
Man verwendet Kalium-tert.butylat in Dimethylformamid.
$R_f$-Wert: 0,36 (Kieselgel; Methylenchlorid/Methanol/konz. Ammoniak = 19:1:0,1)
(6) 8-[4-(1-Benzyloxycarbonyl-4-piperidinyl)-phenyl]-3,9-dimethyl-1-(2-ethoxycarbonyl-ethyl)-xanthin
Man arbeitet in Dimethylformamid unter Verwendung von Acrylsäure-ethylester.
Schmelzpunkt: 208-210 °C
$R_f$-Wert: 0,75 (Kieselgel; Methylenchlorid/Methanol/Eisessig = 19:1:0,1)

## Beispiel 12

### 8-[2-(1-Benzyloxycarbonyl-4-piperidinyl)-ethyl]-1-(4-ethoxycarbonyl-butyl)-3-methyl-xanthin

a) 4-Amino-5-[[2-(1-benzyloxycarbonyl-4-piperidinyl)-ethyl]-carbonylamino]-1-(4-ethoxycarbonyl-butyl)-3-methyl-1H,3H-pyrimidin-2,6-dion
Eine Lösung von 9,9 g 1-Benzyloxycarbonyl-4-(2-ethoxycarbonyl-ethyl)-piperidin, 9,7 g 4,5-Diamino-1-(4-ethoxycarbonyl-butyl)-3-methyl-1H,3H-pyrimidin-2,6-dion und 4,8 ml Triethylamin in 250 ml Dimethylformamid wird mit 11 g O-(1H-1-Benztriazolyl)-N,N,N',N'-tetramethyl-uronium-tetrafluorborat versetzt und 2,5 Stunden bei Raumtemperatur gerührt. Man dampft ein, verrührt den Rückstand mit einer Mischung aus 300 ml Wasser, 200 ml Tetrahydrofuran und 200 ml Ether und engt die organische Phase ein. Man verreibt mit Ether, löst das entstandene Festprodukt nach dem Abfiltrieren in warmem Aceton und fällt mit Ether.
Ausbeute: 12 g (62% der Theorie),
Schmelzpunkt: 114-115 °C
$R_f$-Wert: 0,37 (Kieselgel; Methylenchlorid/Methanol/konz. Ammoniak = 9:1:0,1, nach zweimaliger Entwicklung)
(b) 8-[2-(1-Benzyloxycarbonyl-4-piperidinyl)-ethyl]-1-(4-ethoxycarbonyl-butyl)-3-methyl-xanthin
10 g 4-Amino-5-[[2-(1-benzyloxycarbonyl-4-piperidinyl)-ethyl]-carbonylamino]-1-(4-ethoxycarbonyl-butyl)-3-methyl-1H,3H-pyrimidin-2,6-dion werden 15 Minuten auf 275 °C erhitzt. Man fügt 0,1 g 4-Dimethylamino-pyridin zu und erhitzt weitere 10 Minuten auf 275 °C. Das Produkt wird chromatographisch über Kieselgel gereinigt (Elutionsmittel: Essigester/Ethanol = 9:1).
Ausbeute: 6,4 g (66% der Theorie),
Schmelzpunkt: 99-100 °C
$R_f$-Wert: 0,68 (Kieselgel; Methylenchlorid/Methanol/konz. Ammoniak = 9:1:0,1)
Analog werden folgende Verbindungen erhalten:
(1) 8-[trans-4-(Benzyloxycarbonylamino-methyl)-cyclohexyl]-1-(4-ethoxycarbonyl-butyl)-3-methyl-xanthin
a) 4-Amino-5-[[trans-4-(benzyloxycarbonylamino-methyl)-cyclohexyl]-carbonylamino]-1-(4-ethoxycarbonyl-butyl)-3-methyl-1H,3H-pyrimidin-2,6-dion
Schmelzpunkt: 152-154 °C (sintert ab 149 °C)
$R_f$-Wert: 0,41 (Kieselgel; Methylenchlorid/Methanol/konz. Ammoniak = 9:1:0,1)
b) 8-[trans-4-(Benzyloxycarbonylamino-methyl)-cyclohexyl]-1-(4-ethoxycarbonyl-butyl)-3-methyl-xanthin
Schmelzpunkt: 155-56 °C
$R_f$-Wert: 0,58 (Kieselgel; Methylenchlorid/Methanol = 19:1, nach zweimaliger Entwicklung)
(2) 8-(4-Cyano-phenyl)-1-(4-ethoxycarbonyl-butyl)-9-methyl-xanthin
Man erhitzt in Gegenwart von 4-Dimethylamino-pyridin 10 Minuten auf 250 °C.
Schmelzpunkt: über 250 °C
$R_f$-Wert: 0,56 (Kieselgel; Methylenchlorid/Methanol = 9:1)
(3) 2-(4-Cyano-phenyl)-9-(4-ethoxycarbonyl-butyl)-8,10-dioxo-5,6-dihydro-4H,9H-pyrimido[1,2,3-cd]purin
Man erhitzt 15 Minuten auf 295-300 °C
Schmelzpunkt: 140 °C
$R_f$-Wert: 0,59 (Kieselgel; Methylenchlorid/Methanol/Eisessig = 9:1:0,1)

Beispiel 13

3,9-Dimethyl-1-(4-ethoxycarbonyl-butyl)-8-[4-(N-hydroxyamidino)-phenyl]-xanthin

20,5 g 8-(4-Cyano-phenyl)-3,9-dimethyl-1-(4-ethoxycarbonyl-butyl)-xanthin werden in 1000 ml Methanol suspendiert und mit 10 g Hydroxylamin-hydrochlorid sowie 17,5 ml N,N-Diisopropylethylamin versetzt. Man erhitzt 4 Stunden zum Rückfluß, dampft ein und verreibt den Rückstand mit 300 ml Eiswasser. Das Festprodukt wird nach Trocknung mit 35 ml Methanol und mit Ether gewaschen.
Ausbeute: 19,7 g (89% der Theorie),
Schmelzpunkt: 226-228 ° C
$R_f$-Wert: 0,39 (Kieselgel; Methylenchlorid/Methanol/konz. Ammoniak = 15:1:0,1, nach zweimaliger Entwicklung)

Beispiel 14

5-(2-Methoxycarbonyl-ethenyl)-1-methyl-2-[4-(4-piperidinyl)-phenyl]-benzimidazol

1,7 g 2-[4-(1-tert.Butyloxycarbonyl-4-piperidinyl)-phenyl]-5-(2-methoxycarbonyl-ethenyl)-1-methyl-benzimidazol werden in 10 ml Methylenchlorid gelöst, mit 10 ml Trifluoressigsäure versetzt und 1,5 Stunden bei Raumtemperatur gerührt. Man dampft im Vakuum ein, neutralisiert mit Natriumbicarbonat-Lösung und rührt zwei Stunden bei Raumtemperatur. Das ausgefallene Festprodukt wird abfiltriert und bei 100 ° C im Vakuum getrocknet.
Ausbeute: 1,1 g (82% der Theorie),
Schmelzpunkt: sintert ab 205 ° C
$R_f$-Wert: 0,52 (Reversed Phase Platte RP18; Methanol/5%ige Natriumchloridlösung = 6:4)

Beispiel 15

2-[4-(1-tert.Butyloxycarbonyl-4-piperidinyl)-phenyl]-5-(2-methoxycarbonyl-ethenyl)-1-methyl-benzimidazol

4 g 3-[4-(1-tert.Butyloxycarbonyl-4-piperidinyl)-benzoylamino]-4-methylamino-zimtsäure-methylester werden in 250 ml Eisessig 2 Stunden auf dem Dampfbad erhitzt. Man dampft ein, nimmt mit Essigester auf und wäscht mit Natriumbicarbonat-Lösung. Die Essigester-Phase wird eingedampft und der Rückstand säulenchromatographisch über Kieselgel gereinigt (Elutionsmittel: Essigester/Petrolether = 6:4).
Ausbeute: 1,5 g (40% der Theorie),
Schmelzpunkt: 177-179 ° C
$R_f$-Wert: 0,55 (Kieselgel; Essigester/Petrolether = 7:3)

Beispiel 16

5-(2-Methoxycarbonyl-ethyl)-1-methyl-2-[4-(4-piperidinyl)-phenyl]-benzimidazol

0,7 g 5-(2-Methoxycarbonyl-ethenyl)-1-methyl-2-[4-(4-piperidinyl)-phenyl]-benzimidazol werden in 15 ml Methanol bei Raumtemperatur in Gegenwart von 0,2 g 10%iger Palladiumkohle 3 Stunden mit Wasserstoff von 4 bar behandelt. Der Katalysator wird abfiltriert und das Filtrat eingedampft.
Ausbeute: 0,7 g (100% der Theorie),
Schmelzpunkt: 138-140 ° C
$R_f$-Wert: 0,56 (Reversed Phase Platte RP18; Methanol/5%ige Natriumchloridlösung = 6:4)
Analog wird folgende Verbindung erhalten:
(1) 5-(2-Methoxycarbonyl-ethyl)-1-methyl-2-(4-piperazino-phenyl)-benzimidazol

Beispiel 17

8-(4-Cyano-phenyl)-2-chlor-1-(4-ethoxycarbonyl-butyl)-9-methyl-hypoxanthin

4,5 g 8-(4-Cyano-phenyl)-1-(4-ethoxycarbonyl-butyl)-9-methyl-xanthin werden in 50 ml Phosphoroxychlorid 4 Stunden zum Rückfluß erhitzt. Das Phosphoroxychlorid wird im Vakuum abdestilliert und der Rückstand in 20 ml Methylenchlorid aufgenommen. Man gießt auf Wasser, neutralisiert mit Natriumbicarbonat, extrahiert mit Essigester und reinigt nach dem Eindampfen durch Chromatographie über Kieselgel (Elutionsmittel: Methylenchlorid/Ethanol = 100:1 bis 100:3).
Ausbeute: 2,5 g (53% der Theorie),
$R_f$-Wert: 0,52 (Kieselgel; Essigester/Ethanol = 9:1)

Beispiel 18

8-(4-Amidino-phenyl)-1-(4-ethoxycarbonyl-butyl)-9-methyl-hypoxanthin-hydrochlorid

0,2 g 8-(4-Amidino-phenyl)-2-chlor-1-(4-ethoxycarbonyl-butyl)-9-methyl-hypoxanthin werden in einer Mischung aus 10 ml Ethanol und 5 ml Tetrahydrofuran in Gegenwart von 0,1g 10%iger Palladium/Kohle bei 40°C 2 Stunden mit Wasserstoff von 4 bar behandelt. Man filtriert vom Katalysator ab, dampft ein und verreibt den Rückstand mit Petrolether.
Ausbeute: 0,13 g (65% der Theorie),
$R_f$-Wert: 0,44 (Reversed Phase Platte RP18; Methanol/5%ige Natriumchloridlösung = 6:4)

Beispiel 19

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 20

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 21

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 22

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 23

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 24

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Kondensierte 5-gliedrige Heterocyclen der allgemeinen Formel

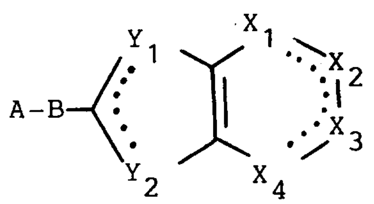

, (I)

in der

A eine Aminoalkyl-, Amidino- oder Guanidinogruppe, in denen jeweils an einem der Stickstoffatome ein Wasserstoffatom durch eine Hydroxy-, Alkyl-, Alkoxycarbonyl- oder Phenylalkoxycarbonylgruppe ersetzt sein kann,

eine gegebenenfalls im Kohlenstoffgerüst durch eine oder zwei Alkylgruppen und am Stickstoffatom durch den Rest $R_a$ substituierte Piperidinylgruppe, in der

$R_a$ ein Wasserstoffatom, eine Alkyl- oder Phenylalkylgruppe, eine Alkoxycarbonylgruppe mit insgesamt 2 bis 5 Kohlenstoffatomen, eine Phenylalkoxycarbonylgruppe oder eine $R_1$-CO-($R_2$CH)-O-CO-Gruppe darstellt, in welcher

$R_1$ eine Alkylgruppe und

$R_2$ ein Wasserstoffatom, eine Alkyl- oder Phenylgruppe darstellen,

und zusätzlich eine $>$CH- Einheit in 4-Stellung durch ein Stickstoffatom ersetzt sein kann,

eine Imidazolylgruppe oder

eine Pyridylgruppe, die falls das heterocyclische System eine Benzoaxazolgruppe darstellt, nicht über die 2-Stellung an den Rest B gebunden sein kann,

B eine geradkettige oder verzweigte Alkylengruppe mit 1 bis 5 Kohlenstoffatomen,

eine -Alkylen-O-, -O-alkylen-, -Alkylen-S-, -S-alkylen-, -Alkylen-$NR_3$-, -$NR_3$-alkylen-, -CO-$NR_3$- oder -$NR_3$-CO-Gruppe, in denen

$R_3$ ein Wasserstoffatom, eine Alkyl- oder Phenylalkylgruppe darstellt,

eine Cyclohexylengruppe oder auch, wenn

A eine gegebenenfalls im Kohlenstoffgerüst durch eine oder zwei Alkylgruppen und am Stickstoffatom durch den Rest $R_a$ substituierte Piperidinylgruppe darstellt, wobei $R_a$ wie oben definiert ist und eine $>$CH-Einheit in 4-Stellung durch ein Stickstoffatom ersetzt sein kann, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe, die im Phenylkern durch Fluor-, Chlor- oder Bromatome, durch Alkyl- oder Alkoxygruppen mono- oder disubstituiert sein kann, wobei die Substituenten gleich oder verschieden sein können,

einer der Reste $X_1$ bis $X_4$ eine F-E-D-C$\lessdot$ oder F-E-D-N$<$ Gruppe, in denen

D eine geradkettige oder verzweigte Alkylengruppe mit 1 bis 6 Kohlenstoffatomen, eine Alkenylengruppe mit 2 bis 6 Kohlenstoffatomen, ein Sauerstoff- oder Schwefelatom, eine -CO-, -SO-, -$SO_2$-, -CO-$NR_3$- oder -$NR_3$-CO-Gruppe oder eine über das Stickstoffatom an den Rest E gebundene -$SO_2$-$NR_3$-Gruppe, wobei $R_3$ wie vorstehend erwähnt definiert ist,

E eine Bindung oder eine geradkettige oder verzweigte Alkylengruppe mit 1 bis 5 Kohlenstoffatomen und

F eine Carboxygruppe, eine Alkoxycarbonylgruppe mit insgesamt 2 bis 7 Kohlenstoffatomen, eine Phenylalkoxycarbonylgruppe, eine Cycloalkoxycarbonyl- oder Cycloalkylalkoxycarbonylgruppe mit jeweils 3 bis 7 Kohlenstoffatomen im Cycloalkylteil, eine Sulfo-, Phosphono-, O-Alkyl-phosphono-, O,O-Dialkyl-phosphono- oder Tetrazol-5-yl-Gruppe darstellen,

ein zweiter der Reste $X_1$ bis $X_4$ ein Stickstoffatom, eine $R_b$-C$\lessdot$ , $R_c$-N$<$ oder Carbonylgruppe, in denen

$R_b$ ein Wasserstoff-, Fluor-, Chlor- oder Bromatom, eine Alkyl-, Hydroxy-, Alkoxy-, Phenylalkoxy-, Amino-, Alkylamino- oder Dialkylaminogruppe, wobei jeder Alkylteil in der vorstehend erwähnten Gruppen jeweils 1 bis 6 Kohlenstoffatome enthalten kann, und

$R_c$ ein Wasserstoffatom, eine gegebenenfalls durch eine Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl-, Pyrrolidino-, Piperidino-, Morpholino-, Piperazino- oder N-Alkyl-piperazinogruppe substituierte Alkylgruppe mit 1 bis 6 Kohlenstoffatomen oder

eine Phenylalkylgruppe mit 1 bis 5 Kohlenstoffatomen im Alkylteil, die im Phenylkern durch Fluor-, Chlor- oder Bromatome, durch Alkyl- oder Alkoxygruppen mono- oder disubstituiert sein kann, wobei die Substituenten gleich oder verschieden sein können, darstellen,

und die verbleibenden Reste der Reste $X_1$ bis $X_4$ jeweils ein Stickstoffatom, eine $R_b$-C$\lessdot$ oder Carbonylgruppe,

wobei $R_b$ wie vorstehend erwähnt definiert ist und nicht mehr als zwei der Reste $X_1$ bis $X_4$ Carbonylgruppen darstellen können und nicht mehr als 3 der Reste $X_1$ bis $X_4$ ein im Ring befindliches Stickstoffatom enthalten können,

einer der Reste $Y_1$ oder $Y_2$ ein Stickstoffatom oder eine Methingruppe und

der andere der Reste $Y_1$ oder $Y_2$ ein Sauerstoffatom oder eine $R_d$-N$<$ Gruppe, in der

$R_d$ ein Wasserstoffatom, eine gegebenenfalls durch eine Hydroxy-, Alkoxy-, Amino-, Alkylamino-, Dialkylamino-, Pyrrolidino-, Piperidino-, Morpholino-, Piperazino- oder N-Alkyl-piperazinogruppe substituierte Alkylgruppe mit 1 bis 6 Kohlenstoffatomen,

eine Phenylalkylgruppe mit 1 bis 5 Kohlenstoffatomen im Alkylteil, die im Phenylkern durch Fluor-, Chlor- oder Bromatome, durch Alkyl- oder Alkoxygruppen mono- oder disubsitutiert sein kann und die Substituenten gleich oder verschieden sein können,

eine Cycloalkylgruppe mit 3 bis 7 Kohlenstoffatomen oder, wenn die Gruppen $R_c$-N$<$ und $R_d$-N$<$ an dasselbe Kohlenstoffatom gebunden sind,

$R_d$ zusammen mit $R_c$ eine geradkettige oder verzweigte Alkylengruppe mit 2 bis 5 Kohlenstoffatomen darstellen,

bedeuten, wobei, soweit nichts anderes erwähnt wurde, die vorstehend erwähnten Alkyl-, Alkylen- und Alkoxyteile jeweils 1 bis 3 Kohlenstoffatome enthalten können,

deren Tautomere, deren Stereoisomere einschließlich deren Gemische und deren Salze.

**2.** Kondensierte 5-gliedrige Heterocyclen der allgemeinen Formel I gemäß Anspruch 1, in der
A eine gegebenenfalls durch eine Hydroxy- oder Alkoxycarbonylgruppe substituierte Amidinogruppe, eine Aminoalkyl- oder Benzyloxycarbonylaminoalkylgruppe,
eine gegebenenfalls im Kohlenstoffgerüst durch eine Alkylgruppe und am Stickstoffatom durch den Rest $R_a$ substituierte Piperidinylgruppe, in der
$R_a$ ein Wasserstoffatom, eine Alkyl-, Benzyl-, Alkoxycarbonyl-, Benzyloxycarbonyl- oder $R_1$-CO-$CH_2$-O-CO-Gruppe darstellt, in welcher
$R_1$ eine Alkylgruppe darstellt,
und zusätzlich eine $>$CH- Einheit in 4-Stellung durch ein Stickstoffatom ersetzt sein kann,
eine Imidazolylgruppe oder
eine Pyridylgruppe, die falls das heterocyclische System eine Benzoxazolgruppe darstellt, nicht über die 2-Stellung an den Rest B gebunden sein kann,
B eine geradkettige oder verzweigte Alkylengruppe mit 1 bis 5 Kohlenstoffatomen,
eine -Alkylen-O-, -O-alkylen-, -Alkylen-S-, -S-alkylen-, -Alkylen-$NR_3$-, -$NR_3$-alkylen-, -CO-$NR_3$- oder -$NR_3$-CO-Gruppe, in denen
$R_3$ ein Wasserstoffatom oder eine Alkylgruppe darstellt,
eine Cyclohexylengruppe oder auch,
wenn A eine gegebenenfalls im Kohlenstoffgerüst durch eine Alkylgruppe und am Stickstoffatom durch den Rest $R_a$ substituierte Piperidinylgruppe darstellt, wobei $R_a$ wie oben definiert ist und eine $>$CH-Einheit in 4-Stellung durch ein Stickstoffatom ersetzt sein kann, oder wenn
$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,
oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,
eine Phenylengruppe,
einer der Reste $X_1$ bis $X_4$ eine F-E-D-C$\ll$ oder F-E-D-N$<$ Gruppe, in denen
D eine geradkettige oder verzweigte Alkylengruppe mit 1 bis 6 Kohlenstoffatomen, eine Alkenylengruppe mit 2 bis 4 Kohlenstoffatomen, ein Sauerstoff- oder Schwefelatom, eine -CO-, -SO-, -$SO_2$-, -CO-$NR_3$- oder -$NR_3$-CO-Gruppe oder eine über das N-Atom an den Rest E gebundene -$SO_2$-$NR_3$-Gruppe, wobei $R_3$ wie vorstehend erwähnt definiert ist,
E eine Bindung oder eine geradkettige oder verzweigte Alkylengruppe mit 1 bis 5 Kohlenstoffatomen und
F eine Carboxygruppe, eine Alkoxycarbonylgruppe mit insgesamt 2 bis 6 Kohlenstoffatomen oder eine Cycloalkoxycarbonylgruppe mit insgesamt 6 bis 8 Kohlenstoffatomen, darstellen,
ein zweiter der Reste $X_1$ bis $X_4$ ein Stickstoffatom, eine $R_b$-C$\ll$ , $R_c$-N$<$ oder Carbonylgruppe, in denen
$R_b$ ein Wasserstoff-, Chlor- oder Bromatom, eine Alkyl-, Hydroxy-, Alkoxy-, Amino-, Alkylamino- oder Dialkylaminogruppe und
$R_c$ ein Wasserstoffatom, eine gegebenenfalls durch eine Hydroxy-, Alkoxy-, Amino-, Alkylamino-, Dialkylamino-, Carboxy-, Alkoxycarbonyl-, Aminocarbonyl-, Pyrrolidino-, Piperidino-, Morpholino-, Piperazino- oder N-Alkyl-piperazinogruppe substituierte Alkylgruppe mit 1 bis 5 Kohlenstoffatomen oder
eine Phenylalkylgruppe mit 1 bis 5 Kohlenstoffatomen im Alkylteil, die im Phenylkern durch Alkoxygruppen mono- oder disubstituiert sein kann, wobei die Substituenten gleich oder verschieden sein können, darstellen,
und die verbleibenden Reste der Reste $X_1$ bis $X_4$ jeweils ein Stickstoffatom, eine $R_b$-C$\ll$ oder Carbonylgruppe, wobei $R_b$ wie vorstehend erwähnt definiert ist und nicht mehr als zwei der Reste $X_1$ bis $X_4$ Carbonylgruppen darstellen können und nicht mehr als 3 der Reste $X_1$ bis $X_4$ ein im Ring befindliches Stickstoffatom enthalten können,
einer der Reste $Y_1$ oder $Y_2$ ein Stickstoffatom und
der andere der Reste $Y_1$ oder $Y_2$ ein Sauerstoffatom oder eine $R_d$-N$<$ Gruppe, in der
$R_d$ ein Wasserstoffatom, eine gegebenenfalls durch eine Hydroxy-, Alkoxy-, Amino-, Alkylamino-, Dialkylamino-, Pyrrolidino-, Piperidino-, Morpholino-, Piperazino- oder N-Alkyl-piperazinogruppe substituierte Alkylgruppe mit 1 bis 5 Kohlenstoffatomen,
eine Phenylalkylgruppe mit 1 bis 5 Kohlenstoffatomen im Alkylteil, die im Phenylkern durch Alkoxygruppen mono- oder disubsitutiert sein kann und die Substituenten gleich oder verschieden sein können,
eine Cycloalkylgruppe mit 3 bis 6 Kohlenstoffatomen oder, wenn die Gruppen $R_c$-N$<$ und $R_d$-N$<$ an dasselbe Kohlenstoffatom gebunden sind,

$R_d$ zusammen mit $R_c$ eine geradkettige oder verzweigte Alkylengruppe mit 2 bis 4 Kohlenstoffatomen darstellen,

bedeuten, wobei, soweit nichts anderes erwähnt wurde, die vorstehend erwähnten Alkyl-, Alkylen- und Alkoxyteile jeweils 1 bis 3 Kohlenstoffatome enthalten können,

deren Tautomere, deren Stereoisomere und deren Salze.

3. Kondensierte 5-gliedrige Heterocyclen der allgemeinen Formel I gemäß Anspruch 1, in der

A eine gegebenenfalls durch eine Hydroxy- oder Alkoxycarbonylgruppe mit insgesamt 2 bis 4 Kohlenstoffatomen substituierte Amidinogruppe, eine Aminoalkyl- oder Benzyloxycarbonylaminoalkylgruppe

eine gegebenenfalls im Kohlenstoffgerüst durch eine Methylgruppe und am Stickstoffatom durch den Rest $R_a$ substituierte Piperidinylgruppe, in der

$R_a$ ein Wasserstoffatom, eine Methyl-, Benzyl-, Methoxycarbonyl, Benzyloxycarbonyl- oder $CH_3$-$CO$-$CH_2$-O-CO-Gruppe darstellt,

und zusätzlich eine $>$CH- Einheit in 4-Stellung durch ein Stickstoffatom ersetzt sein kann,

eine 4-Pyridyl- oder 1-Imidazolylgruppe,

B eine geradkettige oder verzweigte Alkylengruppe mit 1 bis 4 Kohlenstoffatomen,

eine -Alkylen-O-, -O-alkylen-, -Alkylen-S-, -Alkylen-$NR_3$-,-$NR_3$-alkylen- oder -$NR_3$-CO-Gruppe, in denen

der Alkylenteil jeweils 1 oder 2 Kohlenstoffatome enthalten kann sowie der Alkylenteil der -Alkylen-S-Gruppe und das Stickstoffatom der -$NR_3$-CO-Gruppe jeweils mit dem Rest A verbunden ist und

$R_3$ ein Wasserstoffatom oder eine Methylgruppe darstellt,

eine 1,4-Cyclohexylengruppe oder auch,

wenn A eine gegebenenfalls im Kohlenstoffgerüst durch eine Methylgruppe und am Stickstoffatom durch den Rest $R_a$ substituierte Piperidinylgruppe darstellt, wobei $R_a$ wie oben definiert ist und eine $>$CH-Einheit in 4-Stellung durch ein Stickstoffatom ersetzt sein kann, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C$\lll$ oder F-E-D-N$<$ Gruppe, in denen

D eine geradkettige oder verzweigte Alkylengruppe mit 1 bis 6 Kohlenstoffatomen, eine Alkenylengruppe mit 2 bis 4 Kohlenstoffatomen, ein Sauerstoff- oder Schwefelatom, eine -CO-, -SO-, -$SO_2$-, -CO-$NR_3$- oder -$NR_3$-CO-Gruppe oder eine über das Stickstoffatom an den Rest E gebundene -$SO_2$-$NR_3$-Gruppe, wobei $R_3$ wie vorstehend erwähnt definiert ist,

E eine Bindung oder eine geradkettige oder verzweigte Alkylengruppe mit 1 bis 4 Kohlenstoffatomen und

F eine Carboxygruppe, eine Alkoxycarbonylgruppe mit insgesamt 2 bis 6 Kohlenstoffatomen oder eine Cyclohexyloxycarbonylgruppe darstellen,

ein zweiter der Reste $X_1$ bis $X_4$ ein Stickstoffatom, eine $R_c$-N$<$ , $R_b$-C$\lll$ oder Carbonylgruppe, in denen

$R_b$ ein Wasserstoff- oder Chloratom, eine Hydroxy-, Methoxy-, Amino-, Methylamino- oder Dimethylaminogruppe und

$R_c$ ein Wasserstoffatom, eine gegebenenfalls durch eine Methoxy-, Carboxy-, Methoxycarbonyl- oder Aminocarbonylgruppe substituierte Alkylgruppe mit 1 bis 5 Kohlenstoffatomen oder

eine Phenylalkylgruppe mit 1 bis 5 Kohlenstoffatomen im Alkylteil, darstellen,

und die verbleibenden Reste der Reste $X_1$ bis $X_4$ jeweils ein Stickstoffatom, eine $R_b$-C$\lll$ oder Carbonylgruppe, wobei $R_b$ wie vorstehend erwähnt definiert ist und nicht mehr als zwei der Reste $X_1$ bis $X_4$ Carbonylgruppen darstellen können und nicht mehr als zwei der Reste $X_1$ bis $X_4$ ein im Ring befindliches Stickstoffatom enthalten können,

einer der Reste $Y_1$ oder $Y_2$ ein Stickstoffatom und

der andere der Reste $Y_1$ oder $Y_2$ ein Sauerstoffatom oder eine $R_d$-N$<$ Gruppe, in der

$R_d$ ein Wasserstoffatom, eine gegebenenfalls durch eine Hydroxy-, Methoxy-, Amino-, Methylamino-, Dimethylamino-, Morpholino-, Piperazino- oder N-Methyl-piperazinogruppe substituierte Alkylgruppe mit 1 bis 5 Kohlenstoffatomen,

eine Phenylalkylgruppe mit 1 bis 5 Kohlenstoffatomen im Alkylteil, die im Phenylkern durch Methoxygruppen mono- oder disubsitutiert sein kann,

eine Cycloalkylgruppe mit 3 bis 6 Kohlenstoffatomen oder, wenn die Gruppen $R_c$-N$<$ und $R_d$-N$<$

an dasselbe Kohlenstoffatom gebunden sind,

$R_d$ zusammen mit $R_c$ eine geradkettige oder verzweigte Alkylengruppe mit 2 oder 3 Kohlenstoffatomen darstellen,

bedeuten, deren Tautomere, deren Stereoisomere und deren Salze.

**4.** Kondensierte 5-gliedrige Heterocyclen der allgemeinen Formel I gemäß Anspruch 1, in der

A eine Amidino-, Aminoalkyl- oder Benzyloxycarbonylaminoalkylgruppe, in denen der Alkylteil jeweils 1 oder 2 Kohlenstoffatome enthalten kann, oder

eine am Stickstoffatom durch den Rest $R_a$ substituierte Piperidin-4-yl-Gruppe, in der

$R_a$ ein Wasserstoffatom, eine Benzyl- oder Benzyloxycarbonylgruppe darstellt,

B eine geradkettige oder verzweigte Alkylengruppe mit 1 bis 4 Kohlenstoffatomen,

eine -NH-CO-Gruppe, in der das Stickstoffatom der -NH-CO-Gruppe mit dem Rest A verbunden ist,

eine 1,4-Cyclohexylengruppe oder auch,

wenn A eine am Stickstoffatom durch den Rest $R_a$ substituierte Piperidin-4-yl-Gruppe darstellt, wobei $R_a$ wie vorstehend erwähnt definiert ist, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C≪ oder F-E-D-N< -Gruppe, in denen

D eine geradkettige oder verzweigte Alkylengruppe mit 1 bis 4 Kohlenstoffatomen, eine Alkenylengruppe mit 2 oder 3 Kohlenstoffatomen, eine -CO- oder -CO-NH-Gruppe, wobei das Stickstoffatom der -CO-NH-Gruppe mit dem Rest E verknüpft ist,

E eine Bindung oder eine geradkettige oder verzweigte Alkylengruppe mit 2 bis 4 Kohlenstoffatomen und

F eine Carboxygruppe oder eine Alkoxycarbonylgruppe mit insgesamt 2 bis 5 Kohlenstoffatomen darstellen,

ein zweiter der Reste $X_1$ bis $X_4$, eine $R_c$-N< oder $R_b$-C≪ Gruppe, wobei

$R_b$ ein Wasserstoff- oder Chloratom und

$R_c$ ein Wasserstoffatom, eine Methyl- oder Ethylgruppe darstellen,

und die verbleibenden Reste der Reste $X_1$ bis $X_4$ jeweils ein Stickstoffatom, eine H-C≪ oder Carbonylgruppe,

einer der Reste $Y_1$ oder $Y_2$ ein Stickstoffatom und der andere der Reste $Y_1$ oder $Y_2$ eine $R_d$-N< Gruppe, in der

$R_d$ ein Wasserstoffatom oder eine Alkylgruppe mit 1 bis 4 Kohlenstoffatomen oder

$R_d$ zusammen mit $R_c$ eine geradkettige oder verzweigte Alkylengruppe mit 2 oder 3 Kohlenstoffatomen darstellen,

bedeuten, deren Tautomere, deren Stereoisomere und deren Salze.

**5.** Kondensierte 5-gliedrige Heterocyclen der allgemeinen Formel I gemäß Anspruch 1, in der

A eine Amidino- oder Piperidin-4-yl-Gruppe,

B eine Ethylengruppe oder auch, wenn

A eine Piperidin-4-yl-Gruppe darstellt, oder wenn

$X_1$ eine Carbonylgruppe mit der Maßgabe darstellt, daß, falls -D-E-F eine 4-Carboxy-butyl- oder 4-Methoxycarbonyl-butylgruppe darstellt, $X_4$ nicht gleichzeitig eine N-Methyl-iminogruppe bedeutet,

oder, falls -D-E-F eine 2-Carboxyethylaminocarbonyl- oder 2-Methoxycarbonylethylaminocarbonylgruppe darstellt, $X_4$ nicht gleichzeitig ein Stickstoffatom darstellt,

eine Phenylengruppe,

einer der Reste $X_1$ bis $X_4$ eine F-E-D-C≪ oder F-E-D-N< -Gruppe, in denen

D eine geradkettige oder verzweigte Alkylengruppe mit 2 bis 4 Kohlenstoffatomen, eine Ethenylengruppe, eine -CO- oder -CO-NH-Gruppe, wobei das Stickstoffatom der -CO-NH-Gruppe mit dem Rest E verknüpft ist,

E eine Bindung oder eine Ethylengruppe und

F eine Carboxygruppe oder eine Alkoxycarbonylgruppe mit insgesamt 2 bis 5 Kohlenstoffatomen darstellen,

ein zweiter der Reste $X_1$ bis $X_4$, eine $R_c$-N< oder $R_b$-C≪ Gruppe, wobei

$R_b$ ein Wassertoff- oder Chloratom und

$R_c$ ein Wasserstoffatom oder eine Methylgruppe darstellt,
und die verbleibenden Reste der Reste $X_1$ bis $X_4$ jeweils ein Stickstoffatom, eine H-C≪ oder Carbonylgruppe, einer der Reste $Y_1$ oder $Y_2$ ein Stickstoff atom und der andere der Reste $Y_1$ oder $Y_2$ eine $R_d$-N< Gruppe, in der
$R_d$ eine Methylgruppe oder
$R_d$ zusammen mit $R_c$ eine n-Propylengruppe darstellen,
bedeuten, deren Tautomere, deren Stereoisomere und deren Salze.

**6.** Folgende Verbindungen der allgemeinen Formel I gemäß Anspruch 1:
(a) 5-[(2-Carboxy-ethyl)-aminocarbonyl]-1-methyl-2-[2-(4-piperidinyl)-ethyl]-benzimidazol,
(b) 5-[(2-Carboxy-ethyl)-aminocarbonyl]-1-methyl-2-[(4-piperidinyl)-aminocarbonyl]-benzimidazol,
(c) 5-(4-Carboxy-1-oxo-butyl)-1-methyl-2-[2-(4-piperidinyl)-ethyl]-benzimidazol,
(d) 1-(4-Carboxy-butyl)-3-methyl-8-[2-(4-piperidinyl)ethyl]-xanthin,
(e) 1-(4-Carboxy-butyl)-3,9-dimethyl-8-[2-(4-piperidinyl)-ethyl]-xanthin,
(f) 2-(4-Amidino-phenyl)-9-(4-carboxy-butyl)-8,10-dioxo-5,6-dihydro-4H,9H-pyrimido[1,2,3-cd]purin,
(g) 5-[(2-Methoxycarbonyl-ethyl)-aminocarbonyl]-1-methyl-2-[2-(4-piperidinyl)-ethyl]-benzimidazol
und deren Salze.

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der kondensierten 5-gliedrigen Heterocyclen gemäß den Ansprüchen 1 bis 7, dadurch gekennzeichnet, daß
a) zur Herstellung von Verbindungen der allgemeinen Formel I, in der F eine Carboxygruppe darstellt, ein Schutzrest von einer Verbindung der allgemeinen Formel

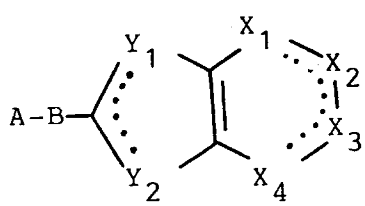

, (II)

in der
A, B, $X_1$ bis $X_4$, $Y_1$ und $Y_2$ mit der Maßgabe wie in den Ansprüchen 1 bis 6 definiert sind, daß F eine Alkoxycarbonyl gruppe mit insgesamt 2 bis 7 Kohlenstoffatomen, eine Phenylalkoxycarbonylgruppe mit 1 bis 3 Kohlenstoffatomen im Alkoxyteil, eine Cycloalkoxycarbonyl- oder Cycloalkylalkoxycarbonylg ruppe mit jeweils 3 bis 7 Kohlenstoffatomen im Cycloalkylteil und 1 bis 3 Kohlenstoffatomen im Alkoxyteil oder eine durch einen abspaltbaren Rest geschützte Carboxylgruppe wie die Trimethylsilyloxycarbonyl-,Methoxybenzyloxycarbonyl-, 2,4-Dimethoxybenzyloxycarbonyl- oder Tetrahydropyranyloxycarbonylgruppe darstellt,mittels Hydrolyse, Hydrogenolyse oder Thermolyse abgespalten wird oder
b) Zur Herstellung von Verbindungen der allgemeinen Formel I, in der $R_a$ ein Wasserstoffatom darstellt, ein Schutzrest von einer Verbindung der allgemeinen Formel

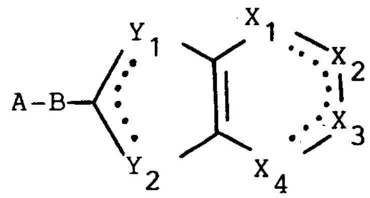

,(III)

in der
A, B, $X_1$ bis $X_4$, $Y_1$ und $Y_2$ mit der Maßgabe wie in den Ansprüchen 1 bis 6 definiert sind, daß $R_a$ eine Alkoxycarbonylgruppe mit insgesamt 2 bis 5 Kohlenstoffatomen, eine Phenylalkoxycarbonylgruppe mit 1 bis 3 Kohlenstoffatomen im Alkoxyteil, eine $R_1$-CO-($R_2$CH)-O-CO-Gruppe, in der $R_1$ und $R_2$ wie in den Ansprüchen 1 bis 6 definiert sind, oder einen abspaltbaren Schutzrest für eine Imino-gruppe darstellt, mittels Hydrolyse, Hydrogenolyse oder Thermolyse abgespalten wird oder

c) zur Herstellung von Verbindungen der allgemeinen Formel I, in der einer der Reste $Y_1$ oder $Y_2$ ein Stickstoffatom und der andere der Reste $Y_1$ oder $Y_2$ eine $R_d$-N$<$ Gruppe oder, wenn die Gruppen $R_c$-N$<$ und $R_d$-N$<$ an dasselbe Kohlenstoffatom gebunden sind, $R_c$ und $R_d$ zusammen auch eine n-Alkylengruppe mit 2 bis 5 Kohlenstoffatomen darstellen, eine gegebenenfalls im Reaktionsgemisch gebildeten Verbindung der allgemeinen Formel

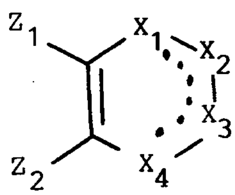

,(IV)

in der
$X_1$ bis $X_4$ wie in den Ansprüchen 1 bis 6 definiert sind, einer der Reste $Z_1$ oder $Z_2$ eine A-B-CO-$NR_{d1}$-Gruppe und der andere der Reste $Z_1$ oder $Z_2$ eine $HNR_{d2}$-Gruppe darstellen, in denen A und B wie in den Ansprüchen 1 bis 6 definiert sind,
einer der Reste $R_{d1}$ oder $R_{d2}$ ein Wasserstoffatom und der andere der Reste $R_{d1}$ oder $R_{d2}$ die für $R_d$ in den Ansprüchen 1 bis 6 erwähnten Bedeutungen besitzt, cyclisiert und gegebenenfalls anschließend von einer so erhaltenen Verbindung ein verwendeter Schutzrest abgespalten wird oder

d) zur Herstellung von Verbindungen der allgemeinen Formel I, in der B eine -$NR_3$-CO-Gruppe darstellt, eine Verbindung der al lgemeinen Formel

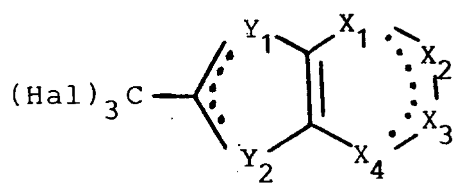

,(V)

in der
$X_1$ bis $X_4$, $Y_1$ und $Y_2$ wie in den Ansprüchen 1 bis 6 definiert sind und
Hal jeweils ein Chlor-, Brom- oder Jodatom darstellen, mit einer Verbindung der allgemeinen Formel

A' - $HNR_3$ ,(VI)

in der
$R_3$ wie in den Ansprüchen 1 bis 6 definiert ist und A' eine gegebenenfalls im Kohlenstoffgerüst durch eine oder zwei Alkylgruppen und am Stickstoffatom durch den Rest $R_a$ substituierte Piperidinylgruppe darstellt, in der $R_a$ mitder Maßgabe wie in den Ansprüchen 1 bis 6 definiert ist, daß $R_a$ mit Ausnahme des Wasserstoffatoms wie in den Ansprüchen 1 bis 6 definiert ist oder einen abspaltbaren Schutzrest für eine Iminogruppe darstellt, umgesetzt und gegebenenfalls anschließend von einer so erhaltenen Verbindung ein verwendeter Schutzrest abgespaltet wird oder
e) zur Herstellung von Verbindungen der allgemeinen Formel I, in der F eine Alkoxycarbonylgruppe mit insgesamt 2 bis 7 Kohle nstoffatomen, eine Phenylalkoxycarbonylgruppe mit 1 bis 3 Kohlenstoffatomen im Alkoxyteil, eine Cycloalkoxycarbonyl- oder Cycloalkylalkoxycarbonylgruppe mit jeweils 3 bis 7 Kohlenstoffatomen im Cycloalkylteil und 1 bis 3 Kohlenstoffatomen im Alkoxyteil darstellt, eine Verbindung der allgemeinen Formel

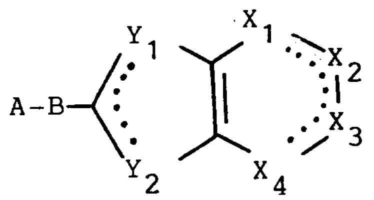

, (VII)

in der
A, B, $X_1$ bis $X_4$, $Y_1$ und $Y_2$ mit der Maßgabe wie in den Ansprüchen 1 bis 6
definiert sind, daß F eine Carboxygruppe oder, falls $Z_3$ eine H ydroxygruppe darstellt, auch eine veresterte Carboxygruppe, z.B. eine Alkoxycarbonylgruppe mit insgesamt 2 bis 5 Kohlenstoffatomen oder eine Phenylalkoxycarbonylgruppe mit 1 bis 3 Kohlenstoffatomen im Alkoxyteil, bedeutet, mit einer Verbindung der allgemeinen Formel

$Z_3$ - $R_4$ ,(VIII)

in der
$R_4$ eine Alkylgruppe mit 1 bis 6 Kohlenstoffatomen, eine Phenylalkylgruppe mit 1 bis 3 Kohlenstoffatomen im Alkylteil, eine Cycloalkyl- oder Cycloalkylalkylgruppe mit jeweils 3 bis 7 Kohlenstoffatomen im Cycloalkylteil und 1 bis 3 Kohlenstoffatomen im Alkylteil und
$Z_3$ eine Hydroxygruppe oder, wenn F eine Carboxygruppe darstellt, eine nukleophile Austrittsgruppe darstellen, umgesetzt wird oder
f) zur Herstellung von Verbindungen der allgemeinen Formel I, in der A eine gegebenenfalls an einem Stickstoffatom durch eine Alkylgruppe mit 1 bis 3 Kohlenstoffatomen substituierte Amidinogruppe darstellt,
eine gegebenenfalls im Reaktionsgemisch gebildete Verbindung der allgemeinen Formel

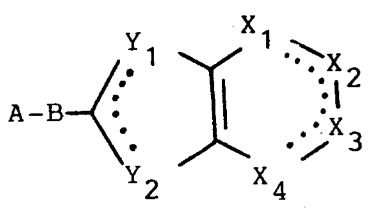

, (IX)

in der
A, B, $X_1$ bis $X_4$, $Y_1$ und $Y_2$ mit der Maßgabe wie in den Ansprüchen 1 bis 6
definiert sind, daß A eine $Z_4$-C(=NH)-Gruppe darstellt, in der $Z_4$ eine Amino-, Alkoxy-, Alkylthio-, Aralkoxy- oder Aralkylthiogruppe darstellt, mit einem Amin der allgemeinen Fo rmel

$R_5$ - $NH_2$, ,(X)

in der
$R_5$ ein Wasserstoffatom oder eine Alkylgruppe mit 1 bis 3 Kohlenstoffatomen bedeutet, oder mit deren Säureadditionssalze n umgesetzt wird oder
g) zur Herstellung einer Verbindung der allgemeinen Formel I, in der A eine Amidino- oder Guanidinogruppe, in denen jeweils an einem der Stickstoffatome ein Wasserstoffatom durch eine Alkyl- oder Alkoxycarbonylgruppe, wobei die vorstehend erwähnten Alkyl- und Alkoxyteile jeweils 1 bis 3 Kohlenstoffatome enthalten können, ersetzt ist, oder eine gegebenenfalls im Kohlenstoffgerüst

durch eine oder zwei Alkylgruppen und am Stickstoffatom durch den Rest $R_a$ substituierte Piperidinylgruppe, in der $R_a$ mit Ausnahme des Wasserstoffatoms wie in den Ansprüchen 1 bis 6 definiert ist, wobei in der Piperidinylgruppe zusätzlich eine >CH-Einheit in 4-Stellung durch ein Stickstoffatom ersetzt sein kann, eine Verbindung der allgemeinen Formel

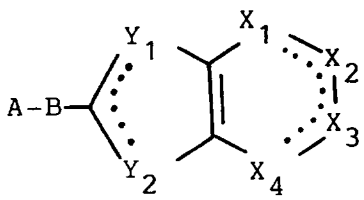

, (XI)

in der
A, B, $X_1$ bis $X_4$, $Y_1$ und $Y_2$ mit der Maßgabe wie in den Ansprüchen 1 bis 6 definiert sind, daß A eine Amidino- oder Guanidinogruppe oder eine gegebenenfalls im Kohlenstoffgerüst durch eine oder zwei Alkylgruppen und am Stickstoffatom unsubstituierte Piperidinylgruppe, wobei in der Piperidinylgruppe zusätzlich eine >CH-Einheit in 4-Stellung durch ein Stickstoffatom ersetzt sein kann darstellt, mit einer Verbindung der allgemeinen Formel

$Z_5$ - $R_6$ ,(XII)

in der
$R_6$ eine Alkoxycarbonylgruppe mit insgesamt 2 bis 5 Kohlenstoffatomen, eine Alkyl-, Phenylalkyl- oder Phenylalkoxycarbonylgruppe mit jeweils 1 bis 3 Kohlenstoffatome im Alkyl- oder Alkoxyteil oder eine $R_1$-CO-($R_2$CH)-O-CO-Gruppe, wobei $R_1$ und $R_2$ wie in den Ansprüchen 1 bis 6 definiert sind, und $Z_5$ eine nukleophile Austrittsgruppe oder, wenn $R_6$ eine Alkyl- oder Phenylalkylgruppe darstellt, $Z_5$ zusammen mit dem Wasserstoffatom der benachbarten Methylengruppe des Restes $R_6$ auch ein Sauerstoffatom darstellen, umgesetzt wird oder
h) zur Herstellung von Verbindungen der allgemeinen Formel I, in der D eine Sulfinyl- oder Sulfonylgruppe darstellt, eine Verbindung der allgemeinen Formel

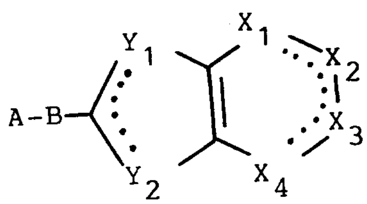

, (XIII)

in der
A, B, $X_1$ bis $X_4$, $Y_1$ und $Y_2$ mit der Maßgabe wie in den Ansprüchen 1 bis 6
definiert sind, daß D ein Schwefelatom oder eine Sulfinylgrupp e darstellt, oxidiert wird oder
i) zur Herstellung von Verbindungen der allgemeinen Formel I, in der einer der Reste $X_1$ bis $X_4$ eine F-E-D-N< Gruppe darstellt, wobei D eine geradkettige oder verzweigte Alkylgruppe mit 1 bis 6 Kohlenstoffatomen oder eine Alkenylgruppe mit 2 bis 6 Kohlenstoffatomen darstellt, eine Verbindung der allgemeinen Formel

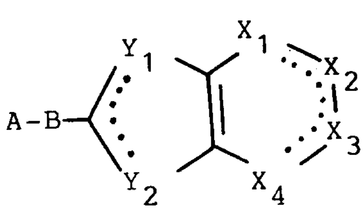

, (XIV)

in der

A, B, $Y_1$, $Y_2$ und $X_1$ bis $X_4$ mit der Maßgabe wie in den Ansprüchen 1 bis 6 definiert sind, daß einer der Reste $X_1$ bis $X_4$ eine H-N< Gruppe darstellt, mit einer Verbindung der allgemeinen Formel

$Z_6$ - D' - E - F ,(XV)

in der

E und F wie in den Ansprüchen 1 bis 6 definiert sind,

D' eine geradkettige oder verzweigte Alkylgruppe mit 1 bis 6 Kohlenstoffatomen oder eine Alkenylgruppe mit 2 bis 6 Kohlenstoffatomen und

$Z_6$ eine Austrittsgruppe oder, falls D' eine unmittelbar an den Rest F gebundene Kohlenstoff-Kohlenstoff-Doppelbindung enthält, auch ein Wasserstoffatom bedeuten, umgesetzt wird oder

j) zur Herstellung von Verbindungen der allgemeinen Formel I, in der D eine Alkylengruppe mit 2 bis 6 Kohlenstoffatomen darstellt, eine Verbindung der allgemeinen Formel

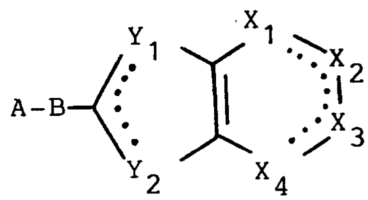

,(XVI)

in der

A, B, $Y_1$, $Y_2$ und $X_1$ bis $X_4$ mit der Maßgabe wie in den Ansprüchen 1 bis 6 definiert sind, daß D eine Alkenylengruppe mit 2 bis 6 Kohlenstoffatomen darstellt, hydriert wird oder

k) zur Herstellung von Verbindungen der allgemeinen Formel I, in der A eine durch eine Hydroxygruppe substituierte Amidinogruppe darstellt, eine Verbindung der allgemeinen Formel

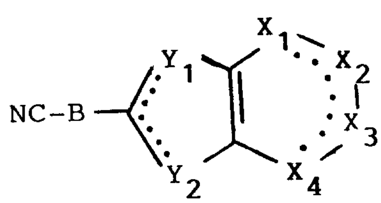

,(XVII)

in der

B, $Y_1$, $Y_2$, und $X_1$ bis $X_4$ wie in den Ansprüchen 1 bis 6 definiert sind, mit Hydroxylamin oder dessen Salzen in Gegenwart einer Base, umgesetzt wird oder

l) zur Herstellung von Verbindungen der allgemeinen Formel I, in der mindestens einer der Reste $X_1$ bis $X_4$ eine Methingruppe darstellt, eine Verbindung der allgemeinen Formel

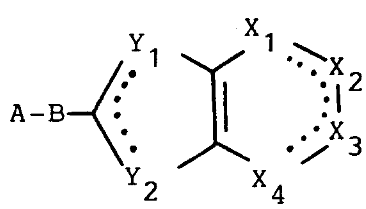

,(XVIII)

in der

A, B, $Y_1$, $Y_2$ und $X_1$ bis $X_4$ mit der Maßgabe wie in den Ansprüchen 1 bis 6 definiert sind, daß mindestens einer der Reste $X_1$ bis $X_4$ eine durch ein Chlor-, Brom- oder Jodatom substituierte Methingruppe darstellt, enthalogeniert wird und

gewünschtenfalls anschließend eine so erhaltene Verbindung der allgemeinen Formel I, in der mindestens einer der Reste $X_1$ bis $X_4$ eine Carbonylgruppe darstellt, mittels Halogenierung in eine entsprechende Verbindung der allgemeinen Formel I, in der mindestens einer der Reste $X_1$ bis $X_4$

eine durch ein Chlor- oder Bromatom substituierte Methingruppe darstellt, übergeführt wird und/oder erforderlichenfalls ein während den Umsetzungen zum Schutze von reaktiven Gruppen verwendeter Schutzrest abgespalten wird und/oder gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.